# EUROPEAN PATENT SPECIFICATION

(11) **EP 2 834 638 B1**
(45) Date of publication and mention of the grant of the patent: **12.02.2020**
(21) Application number: 13772034.8
(22) Date of filing: 02.04.2013
(51) Int. Cl.: G01N 33/68, G01N 33/74

(54) **METHODS FOR DIAGNOSIS AND PROGNOSIS OF SEPSIS**
VERFAHREN ZUR DIAGNOSE UND PROGNOSE VON SEPSIS
PROCÉDÉS DE DIAGNOSTIC ET DE PRONOSTIC D'UN SEPSIS

(30) Priority: 02.04.2012 US 201261619037 P
(43) Date of publication of application: 11.02.2015
(60) Divisional application: 20151008.8
(73) Proprietor: Astute Medical, Inc., San Diego, CA 92121 (US)
(72) Inventor: ANDERBERG, Joseph, Encinitas, CA 92024 (US); GRAY, Jeff, Solana Beach, CA 92075 (US); MCPHERSON, Paul, Encinitas, CA 92024 (US); NAKAMURA, Kevin, Cardiff By The Sea, CA 92007 (US); KAMPF, James, Patrick, San Diego, CA 92130 (US)
(74) Representative: Wilding, James Roger
(86) International application number: PCT/US2013/035032
(87) International publication number: WO 2013/152047

(56) References cited:
- WO-A1-2011/075744
- WO-A1-2011/097539
- WO-A2-2004/043236
- WO-A2-2009/006347
- WO-A2-2009/006347
- S. KIBE ET AL: "Diagnostic and prognostic biomarkers of sepsis in critical care", JOURNAL OF ANTIMICROBIAL CHEMOTHERAPY, vol. 66, no. Supplement 2, 1 April 2011 (2011-04-01), pages ii33-ii40, XP055206151, ISSN: 0305-7453, DOI: 10.1093/jac/dkq523
- CHAMINDIE PUNYADEERA ET AL: "A biomarker panel to discriminate between systemic inflammatory response syndrome and sepsis and sepsis severity", INTERNET CITATION, 1 January 2010 (2010-01-01), pages 1-18, XP002675479, ISSN: 0974-2700 Retrieved from the Internet: URL:http://www.ncbi.nlm.nih.gov/pmc/articl e/PMC2823139/?report=printable [retrieved on 2012-05-08]
- N ONENLI-MUNGAN ET AL: "Growth hormone and insulin-like growth factor 1 levels and their relation to survival in children with bacterial sepsis and septic shock", JOURNAL OF PAEDIATRICS AND CHILD HEALTH, vol. 40, no. 4, 1 April 2004 (2004-04-01), pages 221-226, XP055206171, ISSN: 1034-4810, DOI: 10.1111/j.1440-1754.2004.00342.x
- SANDRA HEESCH ET AL: "Expression of IGFBP7 in acute leukemia is regulated by DNA methylation", CANCER SCIENCE, vol. 102, no. 1, 29 October 2010 (2010-10-29), pages 253-259, XP055206177, ISSN: 1347-9032, DOI: 10.1111/j.1349-7006.2010.01760.x
- RANIA B. GEORGES ET AL: "The insulin-like growth factor binding proteins 3 and 7 are associated with colorectal cancer and liver metastasis", CANCER BIOLOGY & THERAPY, vol. 12, no. 1, 1 July 2011 (2011-07-01), pages 69-79, XP055206175, ISSN: 1538-4047, DOI: 10.4161/cbt.12.1.15719

## Description

This application claims priority to U.S. Provisional Patent Application 61/619,037 filed April 2, 2012.

### BACKGROUND OF THE INVENTION

The following discussion of the background of the invention is merely provided to aid the reader in understanding the invention and is not admitted to describe or constitute prior art to the present invention.

The term "sepsis" has been used to describe a variety of clinical conditions related to systemic manifestations of inflammation accompanied by an infection. Because of clinical similarities to inflammatory responses secondary to non-infectious etiologies, identifying sepsis has been a particularly challenging diagnostic problem. Recently, the American College of Chest Physicians and the American Society of Critical Care Medicine (Bone et al., Chest 101: 1644-53, 1992) published definitions for "Systemic Inflammatory Response Syndrome" (or "SIRS"), which refers generally to a severe systemic response to an infectious or non-infectious insult, and for the related syndromes "sepsis," "severe sepsis," and "septic shock," and extending to multiple organ dysfunction syndrome ("MODS"). These definitions, described below, are intended for each of these phrases for the purposes of the present application.

A systemic inflammatory response leading to a diagnosis of SIRS may be related to both infection and to numerous non-infective etiologies, including burns, pancreatitis, trauma, heat stroke, and neoplasia. While conceptually it may be relatively simple to distinguish between sepsis and non-septic SIRS, no diagnostic tools have been described to unambiguously distinguish these related conditions. *See, e.g.,* Llewelyn and Cohen, Int. Care Med. 27: S10-S32, 2001. For example, because more than 90% of sepsis cases involve bacterial infection, the "gold standard" for confirming infection has been microbial growth from blood, urine, pleural fluid, cerebrospinal fluid, peritoneal fluid, synnovial fluid, sputum, or other tissue specimens. Such culture has been reported, however, to fail to confirm 50% or more of patients exhibiting strong clinical evidence of sepsis. *See, e.g.,* Jaimes et al., Int. Care Med 29: 1368-71, published electronically June 26, 2003.

Thus, despite improvements in the management of critically ill patients, sepsis remains the leading cause of death in such patients. This makes early determination diagnosis vital. The two biomarkers that have been most widely studied and used in patients with sepsis are C-reactive protein (CRP) and procalcitonin (PCT). Levels of both these biomarkers have been demonstrated to be raised in patients with sepsis, but because they lack specificity for sepsis and levels may be raised in other inflammatory diseases, these biomarkers are more useful for ruling out sepsis than for ruling it in, that is, a completely normal value makes a diagnosis of sepsis less likely, but an elevated level may not be due to infection.

Moreover, biomarkers are relevant in clinical practice not only for their ability to diagnose a pathological condition, but also for predicting morbidity and outcome. The ability to assign a severity of illness and outcome likelihood to a sepsis patient is equally vital for triaging of patients and guiding therapeutic decisions. By way of example, development of acute kidney injury (AKI) during sepsis increases patient morbidity, predicts higher mortality, has a significant effect on multiple organ functions, is associated with an increased length of stay in the intensive care unit, and hence consumes considerable healthcare resources. A biomarker able to stratify risk during the first days of admission could differ from one that provides a prediction later in the course of disease. A sequential determination of a biomarker may also be of use in following the acute response to treatment in patients with sepsis.

For example WO 2011/097539 inter alia describes using one or more assays configured to detect a kidney injury marker selected from the group consisting of Coagulation factor VII, CA 19-9, Insulin-like growth factor-binding protein 7, C-X-C motif chemokine 6, and C-C motif chemokine 13 as diagnostic and prognostic biomarkers in renal injuries.

There remains in the art methods and compositions for evaluating sepsis in patients in order to identify onset of disease, and those most at risk for poor outcomes.

### BRIEF SUMMARY OF THE INVENTION

It is an object of the disclosureto provide methods and compositions for evaluating a sepsis patient. As described herein, measurement of one or more biomarkers selected from the group consisting of Alpha-2 macroglobulin, Angiogenin, Angiopoietin-1 receptor, Angiopoietin-related protein 3, Angiopoietin-related protein 4, Angiopoietin-related protein 6, Apolipoprotein A-II, Apolipoprotein C-III, Apolipoprotein(a), Bone morphogenetic protein 7, Cadherin-16, Cadherin-3, Cancer Antigen 15-3, Cancer Antigen 19-9, Carbonic anhydrase 9, Carcinoembryonic antigen-related cell adhesion molecule 1, Carcinoembryonic antigen-related cell adhesion molecule 5, Caspase-3, active, Cathepsin S, C-C motif chemokine 1, C-C motif chemokine 15, C-C motif chemokine 17 , C-C motif chemokine 18, C-C motif chemokine 20, C-C motif chemokine 21, C-C motif chemokine 24, Clusterin, Collagenase 3, C-peptide (Insulin), C-X-C motif chemokine 11, C-X-C motif chemokine 16, Cyclin-dependent kinase inhibitor 1, DDRGK domain-containing protein 1, Endostatin, Epidermal growth factor receptor, Epiregulin, Epithelial cell adhesion molecule, Erythropoietin receptor, Fibroblast growth factor 19, Fibroblast growth factor 23, Galectin-3, Gastric inhibitory polypeptide, Glucagon, Glucagon-like peptide 1 , Glutathione S-transferase P, Heat shock protein beta-1, Heparin-binding growth factor 1, Hepatitis A virus cellular receptor 1, Hepatocyte growth factor receptor, Insulin, Insulin-like growth factor 1 receptor, Insulin-like growth factor-binding protein 6, Insulin-like growth factor-binding protein 7, Interferon alpha-2, Interleukin-20, Interleukin-21, Interleukin-28A, Interleukin-29, Interleukin-33, Involucrin, Islet amyloid polypeptide, Keratin, type II cytoskeletal 1 (Keratin-1,-10 mix), Lymphatic vessel endothelial hyaluronic acid receptor 1, Macrophage metalloelastase, Matrix metalloproteinase-9:Metalloproteinase inhibitor 2 complex, Metalloproteinase inhibitor 3, Myeloid differentiation primary response protein MyD88, Netrin-1, Netrin-4, Neuronal cell adhesion molecule, NF-kappa-B inhibitor alpha, Nidogen-1, Osteocalcin, Pappalysin-1, Poly [ADP-ribose] polymerase 1 (cleaved), Probetacellulin, Prostate-specific antigen, Prostatic acid phosphatase, Protein NOV homolog, Protransforming growth factor alpha, P-selectin glycoprotein ligand 1, Sex hormone-binding globulin, SL cytokine, Tenascin, Thrombospondin-2, Thymic stromal lymphopoietin , Transmembrane glycoprotein NMB, Trefoil factor 3, Tubulointerstitial nephritis antigen, Tumor necrosis factor receptor superfamily member 10B, Tumor necrosis factor receptor superfamily member 8, Versican core protein, Bone morphogenetic protein 7, Carbonic anhydrase 9, Caspase-9, Collagenase 3, Granzyme M, Heparin-binding EGF-like growth factor, Insulin receptor substrate 1, Keratin, type II cytoskeletal 6 (6A, -6B, -6C mix), Myeloid differentiation primary response protein MyD88, SL cytokine, Vascular endothelial growth factor D, Vascular endothelial growth factor receptor 2, and Vascular endothelial growth factor receptor 3 (each referred to herein as a "sepsis biomarker") can be used for diagnosis, prognosis, risk stratification, staging, monitoring, categorizing and determination of further diagnosis and treatment regimens in sepsis patients.

The sepsis biomarkers of the present invention may be used, individually or in panels comprising a plurality of sepsis biomarkers, for identifying a subject suffering from SIRS, sepsis, severe sepsis, septic shock and/or MODS, for distinguishing amongst these conditions, for assigning a risk that a subject at risk for sepsis will progress to sepsis, severe sepsis, septic shock and/or MODS; or for assigning a prognosis to a subject suffering from one or more of these conditions, *etc.*

In a first aspect, the present invention relates to a method of diagnosing SIRS, sepsis, severe sepsis, septic shock, or MODS in a subject, or assigning a prognostic risk for one or more clinical outcomes for a subject suffering from SIRS, sepsis, severe sepsis, septic shock, or MODS, the method comprising:
performing one or more assays configured to detect Insulin-like growth factor-binding protein 7, and optionally further one or more biomarkers selected from the group consisting of Alpha-2 macroglobulin, Angiogenin, Angiopoietin-1 receptor, Angiopoietin-related protein 3, Angiopoietin-related protein 4, Angiopoietin-related protein 6, Apolipoprotein A-II, Apolipoprotein C-III, Apolipoprotein(a), Bone morphogenetic protein 7, Cadherin-16, Cadherin-3, Cancer Antigen 15-3, Cancer Antigen 19-9, Carbonic anhydrase 9, Carcinoembryonic antigen-related cell adhesion molecule 1, Carcinoembryonic antigen-related cell adhesion molecule 5, Caspase-3, active, Cathepsin S, C-C motif chemokine 1, C-C motif chemokine 15, C-C motif chemokine 17 , C-C motif chemokine 18, C-C motif chemokine 20, C-C motif chemokine 21, C-C motif chemokine 24, Clusterin, Collagenase 3, C-peptide (Insulin), C-X-C motif chemokine 11, C-X-C motif chemokine 16, Cyclin-dependent kinase inhibitor 1, DDRGK domain-containing protein 1, Endostatin, Epidermal growth factor receptor, Epiregulin, Epithelial cell adhesion molecule, Erythropoietin receptor, Fibroblast growth factor 19, Fibroblast growth factor 23, Galectin-3, Gastric inhibitory polypeptide, Glucagon, Glucagon-like peptide 1 , Glutathione S-transferase P, Heat shock protein beta-1, Heparin-binding growth factor 1, Hepatitis A virus cellular receptor 1, Hepatocyte growth factor receptor, Insulin, Insulin-like growth factor 1 receptor, Insulin-like growth factor-binding protein 6, Interferon alpha-2, Interleukin-20, Interleukin-21, Interleukin-28A, Interleukin-29, Interleukin-33, Involucrin, Islet amyloid polypeptide, Keratin, type II cytoskeletal 1 (Keratin-1,-10 mix), Lymphatic vessel endothelial hyaluronic acid receptor 1, Macrophage metalloelastase, Matrix metalloproteinase-9:Metalloproteinase inhibitor 2 complex, Metalloproteinase inhibitor 3, Myeloid differentiation primary response protein MyD88, Netrin-1, Netrin-4, Neuronal cell adhesion molecule, NF-kappa-B inhibitor alpha, Nidogen-1, Osteocalcin, Pappalysin-1, Poly [ADP-ribose] polymerase 1 (cleaved), Probetacellulin, Prostate-specific antigen, Prostatic acid phosphatase, Protein NOV homolog, Protransforming growth factor alpha, P-selectin glycoprotein ligand 1, Sex hormone-binding globulin, SL cytokine, Tenascin, Thrombospondin-2, Thymic stromal lymphopoietin , Transmembrane glycoprotein NMB, Trefoil factor 3, Tubulointerstitial nephritis antigen, Tumor necrosis factor receptor superfamily member 10B, Tumor necrosis factor receptor superfamily member 8, Versican core protein, Bone morphogenetic protein 7, Carbonic anhydrase 9, Caspase-9, Collagenase 3, Granzyme M, Heparin-binding EGF-like growth factor, Insulin receptor substrate 1, Keratin, type II cytoskeletal 6 (6A, -6B, -6C mix), Myeloid differentiation primary response protein MyD88, SL cytokine, Vascular endothelial growth factor D, Vascular endothelial growth factor receptor 2, and Vascular endothelial growth factor receptor 3 on a body fluid sample obtained from the sepsis patient for the method of diagnosing or on a urine sample for the method of assessing the prognostic risk to provide one or more assay result(s); and
comparing the assay result(s) in the form of a Insulin-like growth factor-binding protein 7 concentration to a predetermined threshold selected to be indicative of the presence or absence of SIRS, the presence or absence of sepsis, the presence or absence of severe sepsis, the presence or absence of septic shock, the presence or absence of MODS, or
comparing the assay result(s) in the form of a Insulin-like growth factor-binding protein 7 concentration to a predetermined threshold selected to be indicative of the prognostic risk of one or more clinical outcomes, wherein the one or more clinical outcome comprises a prognostic risk of mortality for the subject suffering from or believed to suffer from SIRS, sepsis, severe sepsis, septic shock, or MODS.

Also described are methods for evaluating a sepsis patient or a patient being evaluated for a possible sepsis diagnosis. These methods comprise performing an assay method that is configured to detect one or more biomarkers selected from the group consisting of Alpha-2 macroglobulin, Angiogenin, Angiopoietin-1 receptor, Angiopoietin-related protein 3, Angiopoietin-related protein 4, Angiopoietin-related protein 6, Apolipoprotein A-II, Apolipoprotein C-III, Apolipoprotein(a), Bone morphogenetic protein 7, Cadherin-16, Cadherin-3, Cancer Antigen 15-3, Cancer Antigen 19-9, Carbonic anhydrase 9, Carcinoembryonic antigen-related cell adhesion molecule 1, Carcinoembryonic antigen-related cell adhesion molecule 5, Caspase-3, active, Cathepsin S, C-C motif chemokine 1, C-C motif chemokine 15, C-C motif chemokine 17 , C-C motif chemokine 18, C-C motif chemokine 20, C-C motif chemokine 21, C-C motif chemokine 24, Clusterin, Collagenase 3, C-peptide (Insulin), C-X-C motif chemokine 11, C-X-C motif chemokine 16, Cyclin-dependent kinase inhibitor 1, DDRGK domain-containing protein 1, Endostatin, Epidermal growth factor receptor, Epiregulin, Epithelial cell adhesion molecule, Erythropoietin receptor, Fibroblast growth factor 19, Fibroblast growth factor 23, Galectin-3, Gastric inhibitory polypeptide, Glucagon, Glucagon-like peptide 1 , Glutathione S-transferase P, Heat shock protein beta-1, Heparin-binding growth factor 1, Hepatitis A virus cellular receptor 1, Hepatocyte growth factor receptor, Insulin, Insulin-like growth factor 1 receptor, Insulin-like growth factor-binding protein 6, Insulin-like growth factor-binding protein 7, Interferon alpha-2, Interleukin-20, Interleukin-21, Interleukin-28A, Interleukin-29, Interleukin-33, Involucrin, Islet amyloid polypeptide, Keratin, type II cytoskeletal 1 (Keratin-1,-10 mix), Lymphatic vessel endothelial hyaluronic acid receptor 1, Macrophage metalloelastase, Matrix metalloproteinase-9:Metalloproteinase inhibitor 2 complex, Metalloproteinase inhibitor 3, Myeloid differentiation primary response protein MyD88, Netrin-1, Netrin-4, Neuronal cell adhesion molecule, NF-kappa-B inhibitor alpha, Nidogen-1, Osteocalcin, Pappalysin-1, Poly [ADP-ribose] polymerase 1 (cleaved), Probetacellulin, Prostate-specific antigen, Prostatic acid phosphatase, Protein NOV homolog, Protransforming growth factor alpha, P-selectin glycoprotein ligand 1, Sex hormone-binding globulin, SL cytokine, Tenascin, Thrombospondin-2, Thymic stromal lymphopoietin , Transmembrane glycoprotein NMB, Trefoil factor 3, Tubulointerstitial nephritis antigen, Tumor necrosis factor receptor superfamily member 10B, Tumor necrosis factor receptor superfamily member 8, Versican core protein, Bone morphogenetic protein 7, Carbonic anhydrase 9, Caspase-9, Collagenase 3, Granzyme M, Heparin-binding EGF-like growth factor, Insulin receptor substrate 1, Keratin, type II cytoskeletal 6 (6A, -6B, -6C mix), Myeloid differentiation primary response protein MyD88, SL cytokine, Vascular endothelial growth factor D, Vascular endothelial growth factor receptor 2, and Vascular endothelial growth factor receptor 3, the results of which assay(s) is/are then correlated to the status of the patient. This correlation to status may include correlating the assay result(s) to one or more of diagnosis, risk stratification, prognosis, staging, classifying and monitoring of the sepsis patient as described herein. Thus, the methods utilize one or more sepsis biomarkers disclosed herein for the evaluation of a patient.

In certain embodiments, the methods for evaluating a sepsis patient described herein are methods for risk stratification of the sepsis patient; that is, assigning a likelihood of one or more future changes in health status to the sepsis patient. In these embodiments, the assay result(s) is/are correlated to one or more such future changes. The following are preferred risk stratification embodiments.

In preferred risk stratification embodiments, these methods comprise determining a sepsis patient's risk for future progression to a worsening (or improving) stage within the definition of SIRS. By way of example, the method may comprise assigning a likelihood of progression from SIRS to sepsis; from sepsis to severe sepsis; from sepsis or severe sepsis to septic shock; from sepsis, severe sepsis, or septic shock to MODS. Alternatively, the method may comprise assigning a likelihood of progression from recovery from sepsis; from severe sepsis; from septic shock; from MODS. For example, the measured concentration(s) may each be compared to a threshold value. For a "positive going" sepsis biomarker, an increased likelihood of progression to a worsening stage is assigned to the sepsis patient when the measured concentration is above the threshold, relative to a likelihood assigned when the measured concentration is below the threshold. For a "negative going" sepsis biomarker, an increased likelihood of progressing to a worsening stage is assigned to the sepsis patient when the measured concentration is below the threshold, relative to a likelihood assigned when the measured concentration is above the threshold.

In other preferred risk stratification embodiments, these methods comprise determining a sepsis patient's risk for future reduced renal function, and the assay result(s) is/are correlated to a likelihood of such reduced renal function. For example, the measured concentrations may each be compared to a threshold value. For a "positive going" sepsis biomarker, an increased likelihood of suffering a future reduced renal function is assigned to the sepsis patient when the measured concentration is above the threshold, relative to a likelihood assigned when the measured concentration is below the threshold. For a "negative going" sepsis biomarker, an increased likelihood of future reduced renal function is assigned to the sepsis patient when the measured concentration is below the threshold, relative to a likelihood assigned when the measured concentration is above the threshold.

In yet other preferred risk stratification embodiments, these methods comprise determining a sepsis patient's risk for progression to ARF, and the result(s) is/are correlated to a likelihood of such progression to ARF. For example, the measured concentration(s) may each be compared to a threshold value. For a "positive going" sepsis biomarker, an increased likelihood of progression to ARF is assigned to the sepsis patient when the measured concentration is above the threshold, relative to a likelihood assigned when the measured concentration is below the threshold. For a "negative going" sepsis biomarker, an increased likelihood of progression to ARF is assigned to the sepsis patient when the measured concentration is below the threshold, relative to a likelihood assigned when the measured concentration is above the threshold.

And in other preferred risk stratification embodiments, these methods comprise determining a sepsis patient's outcome risk, and the assay result(s) is/are correlated to a likelihood mortality by the sepsis patient. For example, the measured concentration(s) may each be compared to a threshold value. For a "positive going" sepsis biomarker, an increased likelihood of mortality is assigned to the sepsis patient when the measured concentration is above the threshold, relative to a likelihood assigned when the measured concentration is below the threshold. For a "negative going" sepsis biomarker, an increased likelihood of mortality is assigned to the sepsis patient when the measured concentration is below the threshold, relative to a likelihood assigned when the measured concentration is above the threshold.

In such risk stratification embodiments, preferably the likelihood or risk assigned is that an event of interest is more or less likely to occur within 180 days of the time at which the body fluid sample is obtained from the sepsis patient. In particularly preferred embodiments, the likelihood or risk assigned relates to an event of interest occurring within a shorter time period such as 18 months, 120 days, 90 days, 60 days, 45 days, 30 days, 21 days, 14 days, 7 days, 5 days, 96 hours, 72 hours, 48 hours, 36 hours, 24 hours, 12 hours, or less. A risk at 0 hours of the time at which the body fluid sample is obtained from the sepsis patient is equivalent to diagnosis of a current condition.

In other embodiments, the methods for evaluating status described herein are methods for diagnosis, which refers to identifying a subject suffering from sepsis, severe sepsis, septic shock and/or MODS. In these embodiments, the assay result(s), for example measured concentration(s) of one or more biomarkers selected from the group consisting of Alpha-2 macroglobulin, Angiogenin, Angiopoietin-1 receptor, Angiopoietin-related protein 3, Angiopoietin-related protein 4, Angiopoietin-related protein 6, Apolipoprotein A-II, Apolipoprotein C-III, Apolipoprotein(a), Bone morphogenetic protein 7, Cadherin-16, Cadherin-3, Cancer Antigen 15-3, Cancer Antigen 19-9, Carbonic anhydrase 9, Carcinoembryonic antigen-related cell adhesion molecule 1, Carcinoembryonic antigen-related cell adhesion molecule 5, Caspase-3, active, Cathepsin S, C-C motif chemokine 1, C-C motif chemokine 15, C-C motif chemokine 17 , C-C motif chemokine 18, C-C motif chemokine 20, C-C motif chemokine 21, C-C motif chemokine 24, Clusterin, Collagenase 3, C-peptide (Insulin), C-X-C motif chemokine 11, C-X-C motif chemokine 16, Cyclin-dependent kinase inhibitor 1, DDRGK domain-containing protein 1, Endostatin, Epidermal growth factor receptor, Epiregulin, Epithelial cell adhesion molecule, Erythropoietin receptor, Fibroblast growth factor 19, Fibroblast growth factor 23, Galectin-3, Gastric inhibitory polypeptide, Glucagon, Glucagon-like peptide 1 , Glutathione S-transferase P, Heat shock protein beta-1, Heparin-binding growth factor 1, Hepatitis A virus cellular receptor 1, Hepatocyte growth factor receptor, Insulin, Insulin-like growth factor 1 receptor, Insulin-like growth factor-binding protein 6, Insulin-like growth factor-binding protein 7, Interferon alpha-2, Interleukin-20, Interleukin-21, Interleukin-28A, Interleukin-29, Interleukin-33, Involucrin, Islet amyloid polypeptide, Keratin, type II cytoskeletal 1 (Keratin-1,-10 mix), Lymphatic vessel endothelial hyaluronic acid receptor 1, Macrophage metalloelastase, Matrix metalloproteinase-9:Metalloproteinase inhibitor 2 complex, Metalloproteinase inhibitor 3, Myeloid differentiation primary response protein MyD88, Netrin-1, Netrin-4, Neuronal cell adhesion molecule, NF-kappa-B inhibitor alpha, Nidogen-1, Osteocalcin, Pappalysin-1, Poly [ADP-ribose] polymerase 1 (cleaved), Probetacellulin, Prostate-specific antigen, Prostatic acid phosphatase, Protein NOV homolog, Protransforming growth factor alpha, P-selectin glycoprotein ligand 1, Sex hormone-binding globulin, SL cytokine, Tenascin, Thrombospondin-2, Thymic stromal lymphopoietin , Transmembrane glycoprotein NMB, Trefoil factor 3, Tubulointerstitial nephritis antigen, Tumor necrosis factor receptor superfamily member 10B, Tumor necrosis factor receptor superfamily member 8, Versican core protein, Bone morphogenetic protein 7, Carbonic anhydrase 9, Caspase-9, Collagenase 3, Granzyme M, Heparin-binding EGF-like growth factor, Insulin receptor substrate 1, Keratin, type II cytoskeletal 6 (6A, -6B, -6C mix), Myeloid differentiation primary response protein MyD88, SL cytokine, Vascular endothelial growth factor D, Vascular endothelial growth factor receptor 2, and Vascular endothelial growth factor receptor 3 is/are correlated to the occurrence or nonoccurrence disease. The following are preferred diagnostic embodiments. In various embodiments, the methods comprise relating the assay result(s) to ruling in or out one or more of the following diagnoses: that the subject has at least sepsis; that the subject has at least severe sepsis; that the subject has at least septic shock; that the subject has MODS.

In preferred diagnostic embodiments, these methods comprise distinguishing among SIRS, sepsis, severe sepsis, septic shock and/or MODS. These methods comprise relating the assay result(s) to ruling in or out one or more of the following diagnoses: that the subject has SIRS, but not sepsis, severe sepsis, septic shock, or MODS; that the subject has sepsis, but not severe sepsis, septic shock, or MODS; that the subject has septic shock but not MODS; that the subject has MODS. For a positive going marker, an increased likelihood of the occurrence of a diagnosis is assigned to the patient when the measured concentration is above the threshold (relative to the likelihood assigned when the measured concentration is below the threshold); alternatively, when the measured concentration is below the threshold, an increased likelihood of the nonoccurrence of a diagnosis may be assigned to the patient (relative to the likelihood assigned when the measured concentration is above the threshold). For a negative going marker, an increased likelihood of the occurrence of a diagnosis is assigned to the patient when the measured concentration is below the threshold (relative to the likelihood assigned when the measured concentration is above the threshold); alternatively, when the measured concentration is above the threshold, an increased likelihood of the nonoccurrence of a diagnosis may be assigned to the patient (relative to the likelihood assigned when the measured concentration is below the threshold).

A variety of methods may be used by the skilled artisan to arrive at a desired threshold value for use in these methods. For example, for a positive going marker the threshold value may be determined from a population of SIRS patients not having sepsis by selecting a concentration representing the 75^{th}, 85^{th}, 90^{th}, 95^{th}, or 99^{th} percentile of a sepsis biomarker measured in such SIRS patients. Alternatively, the threshold value may be determined from a "diseased" population of sepsis patients by selecting a concentration representing the 75^{th}, 85^{th}, 90^{th}, 95^{th}, or 99^{th} percentile of a sepsis biomarker measured in such sepsis patients.

Alternatively, the threshold value may be determined from a "diseased" population of sepsis patients having a predisposition for an outcome such as death, worsening disease, AKI, *etc*.), by selecting a concentration representing the 75^{th}, 85^{th}, 90^{th}, 95^{th}, or 99^{th} percentile of a sepsis biomarker measured in such sepsis patients. In another alternative, the threshold value may be determined from a prior measurement of a sepsis biomarker in the same sepsis patient; that is, a temporal change in the level of a sepsis biomarker in the sepsis patient may be used to assign risk to the sepsis patient.

The foregoing discussion is not meant to imply, however, that the sepsis biomarkers disclosed herein must be compared to corresponding individual thresholds. Methods for combining assay results can comprise the use of multivariate logistical regression, loglinear modeling, neural network analysis, n-of-m analysis, decision tree analysis, calculating ratios of markers, *etc.* This list is not meant to be limiting. In these methods, a composite result which is determined by combining individual markers may be treated as if it is itself a marker; that is, a threshold may be determined for the composite result as described herein for individual markers, and the composite result for an individual patient compared to this threshold.

The ability of a particular test to distinguish two populations can be established using ROC analysis. For example, ROC curves established from a "first" subpopulation which has a particular disease (or which is predisposed to some outcome), and a "second" subpopulation which does not have the disease (or is not so predisposed) can be used to calculate a ROC curve, and the area under the curve provides a measure of the quality of the test. Preferably, the tests described herein provide a ROC curve area greater than 0.5, preferably at least 0.6, more preferably 0.7, still more preferably at least 0.8, even more preferably at least 0.9, and most preferably at least 0.95.

In certain aspects, the measured concentration of one or more sepsis biomarkers, or a composite of such markers, may be treated as continuous variables. For example, any particular concentration can be converted into a corresponding probability of existing disease, of a future outcome for the sepsis patient, or mortality, of a SIRS classification, etc. In yet another alternative, a threshold that can provide an acceptable level of specificity and sensitivity in separating a population of sepsis patients into "bins" such as a "first" subpopulation and a "second" subpopulation. A threshold value is selected to separate this first and second population by one or more of the following measures of test accuracy:
an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;
a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;
a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;
at least about 75% sensitivity, combined with at least about 75% specificity;
a positive likelihood ratio (calculated as sensitivity/(1-specificity)) of greater than 1, at least about 2, more preferably at least about 3, still more preferably at least about 5, and most preferably at least about 10; or
a negative likelihood ratio (calculated as (1-sensitivity)/specificity) of less than 1, less than or equal to about 0.5, more preferably less than or equal to about 0.3, and most preferably less than or equal to about 0.1.

The term "about" in the context of any of the above measurements refers to +/- 5% of a given measurement.

Multiple thresholds may also be used to assess a sepsis patient. For example, a "first" subpopulation identified by an existing disease, predisposition to a future outcome for the sepsis patient, predisposition to mortality, etc. , and a "second" subpopulation which is not so predisposed can be combined into a single group. This group is then subdivided into three or more equal parts (known as tertiles, quartiles, quintiles, etc., depending on the number of subdivisions). An odds ratio is assigned to sepsis patients based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to suffer one or more future changes in disease status in comparison to someone in the first tertile. The third tertile is also assigned an odds ratio that is relative to that first tertile.

In certain embodiments, the assay method is an immunoassay. Antibodies for use in such assays will specifically bind a full length sepsis biomarker of interest, and may also bind one or more polypeptides that are "related" thereto, as that term is defined hereinafter. Numerous immunoassay formats are known to those of skill in the art. Preferred body fluid samples are selected from the group consisting of urine, blood, serum, saliva, tears, and plasma.

The foregoing method steps should not be interpreted to mean that the sepsis biomarker assay result(s) is/are used in isolation in the methods described herein. Rather, additional variables or other clinical indicia may be included in the methods described herein. For example, a risk stratification, diagnostic, classification, monitoring, etc. method may combine the assay result(s) with one or more variables measured for the sepsis patient selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, or renal insufficiency, clinical variables (e.g., blood pressure, temperature, respiration rate), risk scores (APACHE score, PREDICT score, TIMI Risk Score for UA/NSTEMI, Framingham Risk Score).

When more than one marker is measured, the individual markers may be measured in samples obtained at the same time, or may be determined from samples obtained at different (e.g., an earlier or later) times. The individual markers may also be measured on the same or different body fluid samples. For example, one sepsis biomarker may be measured in a serum or plasma sample and another sepsis biomarker may be measured in a urine sample. In addition, assignment of a likelihood may combine an individual sepsis biomarker assay result with temporal changes in one or more additional variables.

In various related aspects, devices and kits for performing the methods described herein are disclosed. Suitable kits comprise reagents sufficient for performing an assay for at least one of the described sepsis biomarkers, together with instructions for performing the described threshold comparisons.

In certain embodiments, reagents for performing such assays are provided in an assay device, and such assay devices may be included in such a kit. Preferred reagents can comprise one or more solid phase antibodies, the solid phase antibody comprising antibody that detects the intended biomarker target(s) bound to a solid support. In the case of sandwich immunoassays, such reagents can also include one or more detectably labeled antibodies, the detectably labeled antibody comprising antibody that detects the intended biomarker target(s) bound to a detectable label. Additional optional elements that may be provided as part of an assay device are described hereinafter.

Detectable labels may include molecules that are themselves detectable (e.g., fluorescent moieties, electrochemical labels, ecl (electrochemical luminescence) labels, metal chelates, colloidal metal particles, *etc*.) as well as molecules that may be indirectly detected by production of a detectable reaction product (*e.g*., enzymes such as horseradish peroxidase, alkaline phosphatase, *etc*.) or through the use of a specific binding molecule which itself may be detectable (*e.g*., a labeled antibody that binds to the second antibody, biotin, digoxigenin, maltose, oligohistidine, 2,4-dintrobenzene, phenylarsenate, ssDNA, dsDNA, etc.).

Generation of a signal from the signal development element can be performed using various optical, acoustical, and electrochemical methods well known in the art. Examples of detection modes include fluorescence, radiochemical detection, reflectance, absorbance, amperometry, conductance, impedance, interferometry, ellipsometry, *etc.* In certain of these methods, the solid phase antibody is coupled to a transducer (*e.g*., a diffraction grating, electrochemical sensor, etc) for generation of a signal, while in others, a signal is generated by a transducer that is spatially separate from the solid phase antibody (*e.g*., a fluorometer that employs an excitation light source and an optical detector). This list is not meant to be limiting. Antibody-based biosensors may also be employed to determine the presence or amount of analytes that optionally eliminate the need for a labeled molecule.

### DETAILED DESCRIPTION OF THE INVENTION

The present disclosure relates to methods and compositions for diagnosis, differential diagnosis, risk stratification, monitoring, classifying and determination of treatment regimens in patients diagnosed with, or at risk of, sepsis. In various embodiments, a measured concentration of one or more biomarkers selected from the group consisting of Alpha-2 macroglobulin, Angiogenin, Angiopoietin-1 receptor, Angiopoietin-related protein 3, Angiopoietin-related protein 4, Angiopoietin-related protein 6, Apolipoprotein A-II, Apolipoprotein C-III, Apolipoprotein(a), Bone morphogenetic protein 7, Cadherin-16, Cadherin-3, Cancer Antigen 15-3, Cancer Antigen 19-9, Carbonic anhydrase 9, Carcinoembryonic antigen-related cell adhesion molecule 1, Carcinoembryonic antigen-related cell adhesion molecule 5, Caspase-3, active, Cathepsin S, C-C motif chemokine 1, C-C motif chemokine 15, C-C motif chemokine 17 , C-C motif chemokine 18, C-C motif chemokine 20, C-C motif chemokine 21, C-C motif chemokine 24, Clusterin, Collagenase 3, C-peptide (Insulin), C-X-C motif chemokine 11, C-X-C motif chemokine 16, Cyclin-dependent kinase inhibitor 1, DDRGK domain-containing protein 1, Endostatin, Epidermal growth factor receptor, Epiregulin, Epithelial cell adhesion molecule, Erythropoietin receptor, Fibroblast growth factor 19, Fibroblast growth factor 23, Galectin-3, Gastric inhibitory polypeptide, Glucagon, Glucagon-like peptide 1 , Glutathione S-transferase P, Heat shock protein beta-1, Heparin-binding growth factor 1, Hepatitis A virus cellular receptor 1, Hepatocyte growth factor receptor, Insulin, Insulin-like growth factor 1 receptor, Insulin-like growth factor-binding protein 6, Insulin-like growth factor-binding protein 7, Interferon alpha-2, Interleukin-20, Interleukin-21, Interleukin-28A, Interleukin-29, Interleukin-33, Involucrin, Islet amyloid polypeptide, Keratin, type II cytoskeletal 1 (Keratin-1,-10 mix), Lymphatic vessel endothelial hyaluronic acid receptor 1, Macrophage metalloelastase, Matrix metalloproteinase-9:Metalloproteinase inhibitor 2 complex, Metalloproteinase inhibitor 3, Myeloid differentiation primary response protein MyD88, Netrin-1, Netrin-4, Neuronal cell adhesion molecule, NF-kappa-B inhibitor alpha, Nidogen-1, Osteocalcin, Pappalysin-1, Poly [ADP-ribose] polymerase 1 (cleaved), Probetacellulin, Prostate-specific antigen, Prostatic acid phosphatase, Protein NOV homolog, Protransforming growth factor alpha, P-selectin glycoprotein ligand 1, Sex hormone-binding globulin, SL cytokine, Tenascin, Thrombospondin-2, Thymic stromal lymphopoietin , Transmembrane glycoprotein NMB, Trefoil factor 3, Tubulointerstitial nephritis antigen, Tumor necrosis factor receptor superfamily member 10B, Tumor necrosis factor receptor superfamily member 8, Versican core protein, Bone morphogenetic protein 7, Carbonic anhydrase 9, Caspase-9, Collagenase 3, Granzyme M, Heparin-binding EGF-like growth factor, Insulin receptor substrate 1, Keratin, type II cytoskeletal 6 (6A, -6B, -6C mix), Myeloid differentiation primary response protein MyD88, SL cytokine, Vascular endothelial growth factor D, Vascular endothelial growth factor receptor 2, and Vascular endothelial growth factor receptor 3 or one or more markers related thereto, are correlated to the status of the sepsis patient. As described herein, measurement of one or more sepsis biomarkers described herein may be used, individually or in panels comprising a plurality of sepsis biomarkers, for identifying a subject suffering from SIRS, sepsis, severe sepsis, septic shock and/or MODS, for distinguishing amongst these conditions, or for assigning a prognosis to a subject suffering from one or more of these conditions, *etc.*

For purposes of this document, the following definitions apply:
As used herein, "SIRS" refers to a condition that exhibits two or more of the following:
a temperature > 38°C or < 36°C;
a heart rate of > 90 beats per minute (tachycardia);
a respiratory rate of > 20 breaths per minute (tachypnea) or a PₐCO₂ < 4.3 kPa; and
a white blood cell count > 12,000 per mm³, < 4,000 per mm³, or > 10% immature (band) forms.

As used herein, "Sepsis" refers to SIRS, further accompanied by a clinically evident or microbiologically confirmed infection. This infection may be bacterial, fungal, parasitic, or viral.

As used herein, "Severe sepsis" refers to a subset of sepsis patients, in which sepsis is further accompanied by organ hypoperfusion made evident by at least one sign of organ dysfunction such as hypoxemia, oliguria, metabolic acidosis, or altered cerebral function.

As used herein, "Septic shock" refers to a subset of severe sepsis patients, in which severe sepsis is further accompanied by hypotension, made evident by a systolic blood pressure < 90 mm Hg, or the requirement for pharmaceutical intervention to maintain blood pressure.

As used herein, MODS (multiple organ dysfunction syndrome) is the presence of altered organ function in a patient who is acutely ill such that homeostasis cannot be maintained without intervention. Primary MODS is the direct result of a well-defined insult in which organ dysfunction occurs early and can be directly attributable to the insult itself. Secondary MODS develops as a consequence of a host response and is identified within the context of SIRS.

As used herein, an "injury to renal function" is an abrupt (within 14 days, preferably within 7 days, more preferably within 72 hours, and still more preferably within 48 hours) measurable reduction in a measure of renal function. Such an injury may be identified, for example, by a decrease in glomerular filtration rate or estimated GFR, a reduction in urine output, an increase in serum creatinine, an increase in serum cystatin C, a requirement for renal replacement therapy, *etc.* "Improvement in Renal Function" is an abrupt (within 14 days, preferably within 7 days, more preferably within 72 hours, and still more preferably within 48 hours) measurable increase in a measure of renal function. Preferred methods for measuring and/or estimating GFR are described hereinafter.

As used herein, "reduced renal function" is an abrupt (within 14 days, preferably within 7 days, more preferably within 72 hours, and still more preferably within 48 hours) reduction in kidney function identified by an absolute increase in serum creatinine of greater than or equal to 0.1 mg/dL (≥ 8.8 µmol/L), a percentage increase in serum creatinine of greater than or equal to 20% (1.2-fold from baseline), or a reduction in urine output (documented oliguria of less than 0.5 ml/kg per hour).

As used herein, "acute renal failure" or "ARF" is an abrupt (within 14 days, preferably within 7 days, more preferably within 72 hours, and still more preferably within 48 hours) reduction in kidney function identified by an absolute increase in serum creatinine of greater than or equal to 0.3 mg/dl (≥ 26.4 µmol/l), a percentage increase in serum creatinine of greater than or equal to 50% (1. 5-fold from baseline), or a reduction in urine output (documented oliguria of less than 0.5 ml/kg per hour for at least 6 hours). This term is synonymous with "acute kidney injury" or "AKI."

As used herein, the term "relating a signal to the presence or amount" of an analyte reflects the following understanding. Assay signals are typically related to the presence or amount of an analyte through the use of a standard curve calculated using known concentrations of the analyte of interest. As the term is used herein, an assay is "configured to detect" an analyte if an assay can generate a detectable signal indicative of the presence or amount of a physiologically relevant concentration of the analyte. Because an antibody epitope is on the order of 8 amino acids, an immunoassay configured to detect a marker of interest will also detect polypeptides related to the marker sequence, so long as those polypeptides contain the epitope(s) necessary to bind to the antibody or antibodies used in the assay. The term "related marker" as used herein with regard to a biomarker such as one of the sepsis biomarkers described herein refers to one or more fragments, variants, etc., of a particular marker or its biosynthetic parent that may be detected as a surrogate for the marker itself or as independent biomarkers. The term also refers to one or more polypeptides present in a biological sample that are derived from the biomarker precursor complexed to additional species, such as binding proteins, receptors, heparin, lipids, sugars, *etc.*

In this regard, the skilled artisan will understand that the signals obtained from an immunoassay are a direct result of complexes formed between one or more antibodies and the target biomolecule (*i.e.,* the analyte) and polypeptides containing the necessary epitope(s) to which the antibodies bind. While such assays may detect the full length biomarker and the assay result be expressed as a concentration of a biomarker of interest, the signal from the assay is actually a result of all such "immunoreactive" polypeptides present in the sample. Expression of biomarkers may also be determined by means other than immunoassays, including protein measurements (such as dot blots, western blots, chromatographic methods, mass spectrometry, *etc*.) and nucleic acid measurements (mRNA quatitation). This list is not meant to be limiting. With regard to biomarkers which exist in one form as type-I, type-II, or GPI-anchored membrane proteins, such membrane proteins typically comprise a substantial extracellular domain, some or all of which can be detected as soluble forms present in aqueous samples such as blood, serum, plasma, urine, etc., either as cleavage products or as splice variants which delete an effective membrane spanning domain. Preferred assays detect soluble forms of these biomarkers.

The term "positive going" marker as that term is used herein refer to a marker that is determined to be elevated in sepsis patients suffering from a disease or condition, relative to sepsis patients not suffering from that disease or condition. The term "negative going" marker as that term is used herein refer to a marker that is determined to be reduced in sepsis patients suffering from a disease or condition, relative to sepsis patients not suffering from that disease or condition.

The term "subject" as used herein refers to a human or non-human organism. Thus, the methods and compositions described herein are applicable to both human and veterinary disease. Further, while a subject is preferably a living organism, the invention described herein may be used in post-mortem analysis as well. Preferred subjects are humans, and most preferably "patients," which as used herein refers to living humans that are receiving medical care for a disease or condition. This includes persons with no defined illness who are being investigated for signs of pathology. A "sepsis patient" is a patient suffering from sepsis.

Preferably, an analyte such as a sepsis biomarker is measured in a sample. Such a sample may be obtained from a patient, such as a sepsis patient. Preferred samples are body fluid samples.

The term "body fluid sample" as used herein refers to a sample of bodily fluid obtained for the purpose of diagnosis, prognosis, classification or evaluation of a sepsis patient of interest, such as a patient or transplant donor. In certain embodiments, such a sample may be obtained for the purpose of determining the outcome of an ongoing condition or the effect of a treatment regimen on a condition. Preferred body fluid samples include blood, serum, plasma, cerebrospinal fluid, urine, saliva, sputum, and pleural effusions. In addition, one of skill in the art would realize that certain body fluid samples would be more readily analyzed following a fractionation or purification procedure, for example, separation of whole blood into serum or plasma components.

The term "diagnosis" as used herein refers to methods by which the skilled artisan can estimate and/or determine the probability ("a likelihood") of whether or not a patient is suffering from a given disease or condition. In the case of the present invention, "diagnosis" includes using the results of an assay, most preferably an immunoassay, for a sepsis biomarker of the present invention, optionally together with other clinical characteristics, to arrive at a diagnosis (that is, the occurrence or nonoccurrence) of a disease or condition. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the sepsis patient relative to a measured level on the other side of the predetermined diagnostic threshold.

Similarly, a prognostic risk signals a probability ("a likelihood") that a given course or outcome will occur. A level or a change in level of a prognostic indicator, which in turn is associated with an increased probability of morbidity (e.g., worsening sepsis or death) is referred to as being "indicative of an increased likelihood" of an adverse outcome in a patient.

### Sepsis Biomarkers

The following table provides a list of the sepsis biomarkers disclosed herein, together with the Swiss-Prot entry number for the human precursor.

| SwissProtNum | Preferred Name |
|---|---|
| P01023 | Alpha-2 macroglobulin |
| P03950 | Angiogenin |
| Q02763 | Angiopoietin-1 receptor |
| Q9Y5C1 | Angiopoietin-related protein 3 |
| Q9BY76 | Angiopoietin-related protein 4 |
| Q8NI99 | Angiopoietin-related protein 6 |
| P02652 | Apolipoprotein A-II |
| P02656 | Apolipoprotein C-III |
| P08519 | Apolipoprotein(a) |
| P18075 | Bone morphogenetic protein 7 |
| O75309 | Cadherin-16 |
| P22223 | Cadherin-3 |
| - | Cancer Antigen 15-3 |
| - | Cancer Antigen 19-9 |
| Q16790 | Carbonic anhydrase 9 |
| P13688 | Carcinoembryonic antigen-related cell adhesion molecule 1 |
| P06731 | Carcinoembryonic antigen-related cell adhesion molecule 5 |
| P42574 | Caspase-3, active |
| P25774 | Cathepsin S |
| P22362 | C-C motif chemokine 1 |
| Q16663 | C-C motif chemokine 15 |
| Q92583 | C-C motif chemokine 17 |
| P55774 | C-C motif chemokine 18 |
| P78556 | C-C motif chemokine 20 |
| O00585 | C-C motif chemokine 21 |
| 000175 | C-C motif chemokine 24 |
| P10909 | Clusterin |
| P45452 | Collagenase 3 |
| P01308 | C-peptide (Insulin) |
| 014625 | C-X-C motif chemokine 11 |
| Q9H2A7 | C-X-C motif chemokine 16 |
| P38936 | Cyclin-dependent kinase inhibitor 1 |
| Q96HY6 | DDRGK domain-containing protein 1 |
| P39060 | Endostatin |
| P00533 | Epidermal growth factor receptor |
| 014944 | Epiregulin |
| P16422 | Epithelial cell adhesion molecule |
| P19235 | Erythropoietin receptor |
| O95750 | Fibroblast growth factor 19 |
| Q9GZV9 | Fibroblast growth factor 23 |
| P17931 | Galectin-3 |
| P09681 | Gastric inhibitory polypeptide |
| P01275 | Glucagon |
| P01275 | Glucagon-like peptide 1 |
| P09211 | Glutathione S-transferase P |
| P04792 | Heat shock protein beta-1 |
| P05230 | Heparin-binding growth factor 1 |
| Q96D42 | Hepatitis A virus cellular receptor 1 |
| P08581 | Hepatocyte growth factor receptor |
| P01308 | Insulin |
| P08069 | Insulin-like growth factor 1 receptor |
| P24592 | Insulin-like growth factor-binding protein 6 |
| Q16270 | Insulin-like growth factor-binding protein 7 |
| P01563 | Interferon alpha-2 |
| Q9NYY1 | Interleukin-20 |
| Q9HBE4 | Interleukin-21 |
| Q8IZJ0 | Interleukin-28A |
| Q8IU54 | Interleukin-29 |
| O95760 | Interleukin-33 |
| P07476 | Involucrin |
| P10997 | Islet amyloid polypeptide |
| P04264;P13645 | Keratin, type II cytoskeletal 1 (Keratin-1,-10 mix) |
| Q9Y5Y7 | Lymphatic vessel endothelial hyaluronic acid receptor 1 |
| P39900 | Macrophage metalloelastase |
| P14780;P16035 | Matrix metalloproteinase-9:Metalloproteinase inhibitor 2 |
| complex | |
| P35625 | Metalloproteinase inhibitor 3 |
| Q99836 | Myeloid differentiation primary response protein MyD88 |
| 095631 | Netrin-1 |
| Q9HB63 | Netrin-4 |
| Q92823 | Neuronal cell adhesion molecule |
| P25963 | NF-kappa-B inhibitor alpha |
| P14543 | Nidogen-1 |
| P02818 | Osteocalcin |
| Q13219 | Pappalysin-1 |
| P09874 | Poly [ADP-ribose] polymerase 1 (cleaved) |
| P35070 | Probetacellulin |
| P07288 | Prostate-specific antigen |
| P15309 | Prostatic acid phosphatase |
| P48745 | Protein NOV homolog |
| P01135 | Protransforming growth factor alpha |
| Q14242 | P-selectin glycoprotein ligand 1 |
| P04278 | Sex hormone-binding globulin |
| P49711 | SL cytokine |
| P24821 | Tenascin |
| P35442 | Thrombospondin-2 |
| Q969D9 | Thymic stromal lymphopoietin |
| Q14956 | Transmembrane glycoprotein NMB |
| Q07654 | Trefoil factor 3 |
| Q9UJW2 | Tubulointerstitial nephritis antigen |
| 014763 | Tumor necrosis factor receptor superfamily member 10B |
| P28908 | Tumor necrosis factor receptor superfamily member 8 |
| P13611 | Versican core protein |
| P18075 | Bone morphogenetic protein 7 |
| Q16790 | Carbonic anhydrase 9 |
| P55211 | Caspase-9 |
| P45452 | Collagenase 3 |
| P51124 | Granzyme M |
| Q99075 | Heparin-binding EGF-like growth factor |
| P35568 | Insulin receptor substrate 1 |
| P02538;P04259;P48668 | Keratin, type II cytoskeletal 6 (6A, -6B, -6C mix) |
| Q99836 | Myeloid differentiation primary response protein MyD88 |
| P49771 | SL cytokine |
| O43915 | Vascular endothelial growth factor D |
| P35968 | Vascular endothelial growth factor receptor 2 |
| P35916 | Vascular endothelial growth factor receptor 3 |

### Marker Assays

In general, immunoassays involve contacting a sample containing or suspected of containing a biomarker of interest with at least one antibody that specifically binds to the biomarker. A signal is then generated indicative of the presence or amount of complexes formed by the binding of polypeptides in the sample to the antibody. The signal is then related to the presence or amount of the biomarker in the sample. Numerous methods and devices are well known to the skilled artisan for the detection and analysis of biomarkers. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, and The Immunoassay Handbook, David Wild, ed. Stockton Press, New York, 1994.

The assay devices and methods known in the art can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. *See, e.g.,* U.S. Patents 5,631,171; and 5,955,377. One skilled in the art also recognizes that robotic instrumentation including but not limited to Beckman ACCESS®, Abbott AXSYM®, Roche ELECSYS®, Dade Behring STRATUS® systems are among the immunoassay analyzers that are capable of performing immunoassays. But any suitable immunoassay may be utilized, for example, enzyme-linked immunoassays (ELISA), radioimmunoassays (RIAs), competitive binding assays, and the like.

Antibodies or other polypeptides may be immobilized onto a variety of solid supports for use in assays. Solid phases that may be used to immobilize specific binding members include include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

Biological assays require methods for detection, and one of the most common methods for quantitation of results is to conjugate a detectable label to a protein or nucleic acid that has affinity for one of the components in the biological system being studied. Detectable labels may include molecules that are themselves detectable (*e.g.,* fluorescent moieties, electrochemical labels, metal chelates, *etc*.) as well as molecules that may be indirectly detected by production of a detectable reaction product (*e.g.,* enzymes such as horseradish peroxidase, alkaline phosphatase, *etc*.) or by a specific binding molecule which itself may be detectable (e.g., biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homo functional cross-linkers (containing identical reactive groups) and heterofunctional cross-linkers (containing non-identical reactive groups). Homobifunctional cross-linkers that couple through amines, sulfhydryls or react non- specifically are available from many commercial sources. Maleimides, alkyl and aryl halides, alpha-haloacyls and pyridyl disulfides are thiol reactive groups. Maleimides, alkyl and aryl halides, and alpha-haloacyls react with sulfhydryls to form thiol ether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

In certain aspects, kits for the analysis of the described sepsis biomarkers are disclosed. The kit comprises reagents for the analysis of at least one test sample which comprise at least one antibody that binds a sepsis biomarker. The kit can also include devices and instructions for performing one or more of the diagnostic and/or prognostic correlations described herein. Preferred kits will comprise an antibody pair for performing a sandwich assay, or a labeled species for performing a competitive assay, for the analyte. Preferably, an antibody pair comprises a first antibody conjugated to a solid phase and a second antibody conjugated to a detectable label, wherein each of the first and second antibodies bind a sepsis biomarker. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits.

### Antibodies

The term "antibody" as used herein refers to a peptide or polypeptide derived from, modeled after or substantially encoded by an immunoglobulin gene or immunoglobulin genes, or fragments thereof, capable of specifically binding an antigen or epitope. See, e.g. Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CHI domains; (ii) a F(ab')2 fragment, a bivalent fragment comprising two Fab fragments linked by a disulfide bridge at the hinge region; (iii) a Fd fragment consisting of the VH and CHI domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

Antibodies used in the immunoassays described herein preferably specifically bind to a sepsis biomarker disclosed herein. The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In preferred embodiments, Preferred antibodies bind with affinities of at least about 10⁷ M⁻¹, and preferably between about 10⁸ M⁻¹ to about 10⁹ M⁻¹, about 10⁹ M⁻¹ to about 10¹⁰ M⁻¹, or about 10¹⁰ M⁻¹ to about 10¹² M⁻¹.

Affinity is calculated as K_{d} = kₒₙ/k_{off} (k_{off} is the dissociation rate constant, Kₒₙ is the association rate constant and K_{d} is the equilibrium constant). Affinity can be determined at equilibrium by measuring the fraction bound (r) of labeled ligand at various concentrations (c). The data are graphed using the Scatchard equation: r/c = K(n-r): where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Numerous publications discuss the use of phage display technology to produce and screen libraries of polypeptides for binding to a selected analyte. *See, e.g,* Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See, e.g.,* U.S. Patent No. 6,057,098.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an antimouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (e.g., in sandwich assays) may interfere with one another sterically, etc., assay performance of an antibody may be a more important measure than absolute affinity and specificity of an antibody.

While the present application describes antibody-based binding assays in detail, alternatives to antibodies as binding species in assays are well known in the art. These include receptors for a particular target, aptamers, etc. Aptamers are oligonucleic acid or peptide molecules that bind to a specific target molecule. Aptamers are usually created by selecting them from a large random sequence pool, but natural aptamers also exist. High-affinity aptamers containing modified nucleotides can confer improved characteristics on the ligand, such as improved in vivo stability or improved delivery characteristics. Examples of such modifications include chemical substitutions at the ribose and/or phosphate and/or base positions, and may include amino acid side chain functionalities.

### Assay Correlations

The term "correlating" as used herein in reference to the use of biomarkers refers to comparing the presence or amount of the biomarker(s) in a patient to its presence or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

Selecting a diagnostic threshold involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. On the other hand, in situations where treatment options are less effective and more risky, clinicians often need a higher degree of diagnostic certainty. Thus, cost/benefit analysis is involved in selecting a diagnostic threshold.

Suitable thresholds may be determined in a variety of ways. For example, one recommended diagnostic threshold for the diagnosis of acute myocardial infarction using cardiac troponin is the 97.5th percentile of the concentration seen in a normal population. Another method may be to look at serial samples from the same patient, where a prior "baseline" result is used to monitor for temporal changes in a biomarker level.

Population studies may also be used to select a decision threshold. Reciever Operating Characteristic ("ROC") arose from the field of signal dectection therory developed during World War II for the analysis of radar images, and ROC analysis is often used to select a threshold able to best distinguish a "diseased" subpopulation from a "nondiseased" subpopulation. A false positive in this case occurs when the person tests positive, but actually does not have the disease. A false negative, on the other hand, occurs when the person tests negative, suggesting they are healthy, when they actually do have the disease. To draw a ROC curve, the true positive rate (TPR) and false positive rate (FPR) are determined as the decision threshold is varied continuously. Since TPR is equivalent with sensitivity and FPR is equal to 1 - specificity, the ROC graph is sometimes called the sensitivity vs (1 - specificity) plot. A perfect test will have an area under the ROC curve of 1.0; a random test will have an area of 0.5. A threshold is selected to provide an acceptable level of specificity and sensitivity.

In this context, "diseased" is meant to refer to a population having one characteristic (the presence of a disease or condition or the occurrence of some outcome) and "nondiseased" is meant to refer to a population lacking the characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold comparisons, other methods for correlating assay results to a patient classification (occurrence or nonoccurrence of disease, likelihood of an outcome, etc.) include decision trees, rule sets, Bayesian methods, and neural network methods. These methods can produce probability values representing the degree to which a sepsis patient belongs to one classification out of a plurality of classifications.

Measures of test accuracy may be obtained as described in Fischer et al., Intensive Care Med. 29: 1043-51, 2003, and used to determine the effectiveness of a given biomarker. These measures include sensitivity and specificity, predictive values, likelihood ratios, diagnostic odds ratios, and ROC curve areas. The area under the curve ("AUC") of a ROC plot is equal to the probability that a classifier will rank a randomly chosen positive instance higher than a randomly chosen negative one. The area under the ROC curve may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1

Additional clinical indicia may be combined with the sepsis biomarker assay result(s). Other clinical indicia which may be combined with the sepsis biomarker assay result(s) includes demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, or renal insufficiency), risk scores (APACHE score, PREDICT score, TIMI Risk Score for UA/NSTEMI, Framingham Risk Score), a urine total protein measurement, a glomerular filtration rate, an estimated glomerular filtration rate, a urine production rate, a serum or plasma creatinine concentration, a renal papillary antigen 1 (RPA1) measurement; a renal papillary antigen 2 (RPA2) measurement; a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, and/or a renal failure index calculated as urine sodium / (urine creatinine / plasma creatinine).

Combining assay results/clinical indicia in this manner can comprise the use of multivariate logistical regression, loglinear modeling, neural network analysis, n-of-m analysis, decision tree analysis, etc. This list is not meant to be limiting.

### Selecting a Treatment Regimen

Once a diagnosis is obtained, the clinician can readily select a treatment regimen that is compatible with the diagnosis. The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the markers described herein may be used to monitor a course of treatment. For example, improved or worsened prognostic state may indicate that a particular treatment is or is not efficacious.

One skilled in the art readily appreciates that the present invention is well adapted to carry out the objects and obtain the ends and advantages mentioned, as well as those inherent therein. The examples provided herein are representative of preferred embodiments and are exemplary.

### Example 1: Sepsis patient sample collection

The objective of this study is to collect samples from acutely ill patients. Approximately 1900 adults expected to be in the ICU for at least 48 hours will be enrolled. To be enrolled in the study, each patient must meet all of the following inclusion criteria and none of the following exclusion criteria:
Inclusion Criteria
   males and females 18 years of age or older;
   Study population 1: approximately 300 patients that have at least one of:
      shock (SBP < 90 mmHg and/or need for vasopressor support to maintain MAP > 60 mmHg and/or documented drop in SBP of at least 40 mmHg); and
      sepsis;
   Study population 2: approximately 300 patients that have at least one of:
      IV antibiotics ordered in computerized physician order entry (CPOE) within 24 hours of enrollment;
      contrast media exposure within 24 hours of enrollment;
      increased Intra-Abdominal Pressure with acute decompensated heart failure; and
      severe trauma as the primary reason for ICU admission and likely to be hospitalized in the ICU for 48 hours after enrollment;
   Study population 3: approximately 300 patients expected to be hospitalized through acute care setting (ICU or ED) with a known risk factor for acute renal injury (e.g. sepsis, hypotension/shock (Shock = systolic BP < 90 mmHg and/or the need for vasopressor support to maintain a MAP > 60 mmHg and/or a documented drop in SBP > 40 mmHg), major trauma, hemorrhage, or major surgery); and/or expected to be hospitalized to the ICU for at least 24 hours after enrollment;
   Study population 4: approximately 1000 patients that are 21 years of age or older, within 24 hours of being admitted into the ICU, expected to have an indwelling urinary catheter for at least 48 hours after enrollment, and have at least one of the following acute conditions within 24 hours prior to enrollment:
      (i) respiratory SOFA score of ≥ 2 (PaO2/FiO2 <300),
      (ii) cardiovascular SOFA score of ≥ 1 (MAP < 70 mm Hg and/or any vasopressor required).
Exclusion Criteria
   known pregnancy;
   institutionalized individuals;
   previous renal transplantation;
   known acutely worsening renal function prior to enrollment (e.g., any category of RIFLE criteria);
   received dialysis (either acute or chronic) within 5 days prior to enrollment or in imminent need of dialysis at the time of enrollment;
   known infection with human immunodeficiency virus (HIV) or a hepatitis virus;
   meets any of the following:
   (i) active bleeding with an anticipated need for > 4 units PRBC in a day;
   (ii) hemoglobin < 7 g/dL;
   (iii) any other condition that in the physician's opinion would contraindicate drawing serial blood samples for clinical study purposes;
   meets only the SBP < 90 mmHg inclusion criterion set forth above, and does not have shock in the attending physician's or principal investigator's opinion;
After obtaining informed consent, an EDTA anti-coagulated blood sample (10 mL) and a urine sample (25-50 mL) are collected from each patient. Blood and urine samples are then collected at 4 (± 0.5) and 8 (± 1) hours after contrast administration (if applicable); at 12 (± 1), 24 (± 2), 36 (± 2), 48 (± 2), 60 (± 2), 72 (± 2), and 84 (± 2) hours after enrollment, and thereafter daily up to day 7 to day 14 while the subject is hospitalized. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

### Example 2. Immunoassay format

Analytes are measured using standard sandwich enzyme immunoassay techniques. A first antibody which binds the analyte is immobilized in wells of a 96 well polystyrene microplate. Analyte standards and test samples are pipetted into the appropriate wells and any analyte present is bound by the immobilized antibody. After washing away any unbound substances, a horseradish peroxidase-conjugated second antibody which binds the analyte is added to the wells, thereby forming sandwich complexes with the analyte (if present) and the first antibody. Following a wash to remove any unbound antibody-enzyme reagent, a substrate solution comprising tetramethylbenzidine and hydrogen peroxide is added to the wells. Color develops in proportion to the amount of analyte present in the sample. The color development is stopped and the intensity of the color is measured at 540 nm or 570 nm. An analyte concentration is assigned to the test sample by comparison to a standard curve determined from the analyte standards.

### Example 3. Use of analyte as a marker for sepsis

Patients from the intensive care unit (ICU) were classified as positive or negative for sepsis according to clinical diagnosis on each day from enrollment to 6 days after.

Two cohorts were defined as (Cohort 1) patients who had sepsis, and (Cohort 2) patients who did not have sepsis on any day from enrollment to 6 days after (7 days total). Both urine and plasma samples from each patient in Cohorts 1 and 2 were collected on the day of enrollment. The concentrations of the analyte in these samples were measured by standard immunoassay methods using commercially available assay reagents. A receiver operating characteristic (ROC) curve was generated using the concentrations, and the performance of the analyte was assessed by the area under the ROC curve (AUC). The two-tailed *p*-value of the AUC for the analyte was calculated, and if the p-value was less than 0.1, the AUC was considered statistically significant.

### Example 4. Use of analyte as a marker for progression to sepsis

Two cohorts were defined as (Cohort 1) patients who did not have sepsis during the 5 days prior to enrollment but had sepsis on any of the subsequent 7 days (enrollment to 6 days after), and (Cohort 2) patients who did not have sepsis on any of the 5 days prior to enrollment and on any of the subsequent 7 days. The urine and plasma sample collections and analyses using the measured analyte concentrations were the same as in Example 3 above.

### Example 5. Use of analyte as a marker for septic shock

Two cohorts were defined as (Cohort 1) patients who had both sepsis and hypotension on the same day (septic shock), and (Cohort 2) patients who did not have septic shock on any day from enrollment to 6 days after. A patient was classified as having hypotension if his/her systolic blood pressure was below 90mm Hg. The urine and plasma sample collections and analyses using the measured analyte concentrations were the same as in Example 3 above.

### Example 6. Use of analyte as a marker for progression to septic shock

Two cohorts were defined as (Cohort 1) patients who did not have septic shock (see Example 5 for definition) on any of the 5 days prior to enrollment but had septic shock on any of the subsequent 7 days (enrollment to 6 days after), and (Cohort 2) patients who did not have septic shock on any of the 5 days prior to enrollment and on any of the subsequent 7 days. The urine and plasma sample collections and analyses using the measured analyte concentrations were the same as in Example 3 above.

### Example 7. Use of analyte as a marker for death with sepsis

Two cohorts were defined as (Cohort 1) patients who had sepsis on any day from enrollment to 6 days after and died within 30 days after enrollment, and (Cohort 2) patients who did not die and patients who died within 30 days after enrollment but did not have sepsis on any day from enrollment to 6 days after. The urine and plasma sample collections and analyses using the measured analyte concentrations were the same as in Example 3 above.

### Example 8. Use of analyte as a marker for death with septic shock

Two cohorts were defined as (Cohort 1) patients who had septic shock (see Example 5 for definition) on any day from enrollment to 6 days after and died within 30 days after enrollment, and (Cohort 2) patients who did not die and patients who died within 30 days after enrollment but did not have septic shock on any day from enrollment to 6 days after. The urine and plasma sample collections and analyses using the measured analyte concentrations were the same as in Example 3 above.

**Table 1 Use of Biomarkers in Sepsis**

| **Preferred Name** | **1** | **2** | **3** | **4** | **5** | **6** | **7** | **8** | **9** | **10** | **11** | **12** |
|---|---|---|---|---|---|---|---|---|---|---|---|---|
| Alpha-2 macroglobulin | S | | | | | | | | | | S | |
| Angiogenin | | S | | | | S | | | | | | |
| Angiopoietin-1 receptor | | | | | | | S | | | | | |
| Angiopoietin-related protein 3 | | | | | | | | | | | | S |
| Angiopoietin-related protein 4 | | | | | | | | S | | | S | |
| Angiopoietin-related protein 6 | | | | | | | S | S | S | S | | |
| Apolipoprotein A-II | S | S | S | S | | | S | | S | | | |
| Apolipoprotein C-III | S | S | S | S | S | S | S | | | | S | |
| Apolipoprotein(a) | | | | | | | S | | | | | |
| Cadherin-16 | | S | | | S | S | | | | | | |
| Cadherin-3 | | | | S | | | S | S | S | S | S | |
| Cancer Antigen 19-9 | | | | S | | | | | | | | |
| Carcinoembryonic antigen-related cell adhesion | S | | S | | S | | | | S | S | | |
| molecule 1 | | | | | | | | | | | | |
| Carcinoembryonic antigen-related cell adhesion | | | | | S | | S | | S | | S | S |
| molecule 5 | | | | | | | | | | | | |
| Caspase-3, active | | | | | S | S | | | S | | | |
| Cathepsin S | S | | S | S | | | | S | S | S | S | S |
| C-C motif chemokine 1 | S | | S | S | S | | | | | | | |
| C-C motif chemokine 15 | S | S | S | | S | | | | S | | S | S |
| C-C motif chemokine 17 | | | | | | | S | | | S | | |
| C-C motif chemokine 18 | S | S | S | S | S | S | S | | S | | S | S |
| C-C motif chemokine 20 | S | S | S | | | | S | S | | S | | |
| C-C motif chemokine 21 | S | | S | S | | | S | | S | | | |
| C-C motif chemokine 24 | | | | | S | | | | | | | |
| Clusterin | S | | | S | S | | S | | | | S | |
| C-peptide (Insulin) | | | | | S | S | | | | | | |
| C-X-C motif chemokine 11 | S | S | | | S | | S | | S | S | | |
| C-X-C motif chemokine 16 | S | S | | S | | | S | | S | | | |
| Cyclin-dependent kinase inhibitor 1 | S | S | S | S | S | S | | | | | S | S |
| DDRGK domain-containing protein 1 | S | | | | | | | | | | | |
| Epidermal growth factor receptor | S | S | | S | S | | | S | S | S | | |
| Epiregulin | | | | S | | | | | | | | |
| Epithelial cell adhesion molecule | S | | | | | | | | | | | |
| Erythropoietin receptor | S | | | | | | | | S | S | | |
| Fibroblast growth factor 19 | | | | | | | S | S | | S | | |
| Fibroblast growth factor 23 | | S | | | | | | | | | S | |
| Galectin-3 | S | S | | S | | | | | | | | |
| Gastric inhibitory polypeptide | S | S | S | S | | | | | | | | |
| Glucagon-like peptide 1 (GLP-1:aa98-127;aa98-128)) | | | S | | | | S | | | | | |
| Glutathione S-transferase P | | | | | | | | S | | | | |
| Heat shock protein beta-1 | | | | | | | | S | | | | |
| Heparin-binding growth factor 1 | | | | | S | | | | | | | |
| Hepatocyte growth factor receptor | | | | | | S | | S | | S | | S |
| Insulin-like growth factor 1 receptor | | | | | | S | | | | | | |
| Insulin-like growth factor-binding protein 6 | S | | | S | S | S | | S | S | | | |
| Insulin-like growth factor-binding protein 7 | S | S | | | S | S | S | | | | | |
| Interferon alpha-2 | S | | | | | | | | | | | |
| Interleukin-20 | S | S | S | S | S | | | | | | | |
| Interleukin-21 | | | | | | | S | | | | | |
| Interleukin-28A | S | | S | | | | | | | | | |
| Interleukin-29 | S | | S | | | | S | | S | S | | |
| Interleukin-33 | | | | | | | | | S | | | |
| Involucrin | | | | | | | S | | | | S | |
| Islet amyloid polypeptide | | S | | | | | | | | | | |
| Keratin, type II cytoskeletal 1 (Keratin-1,-10 mix) | S | | S | | | | | | | | | |
| Lymphatic vessel endothelial hyaluronic acid receptor | S | | | S | | | S | | S | | | |
| 1 | | | | | | | | | | | | |
| Macrophage metalloelastase | S | | | | S | S | | | | | | |
| Matrix metalloproteinase-9:Metalloproteinase | | | | | S | S | | | | | | |
| inhibitor 2 complex | | | | | | | | | | | | |
| Metalloproteinase inhibitor 3 | S | | | | S | S | S | | S | | | |
| Netrin-1 | | S | | | S | S | | S | S | S | S | S |
| Netrin-4 | | | | | | | S | S | S | S | | |
| Neuronal cell adhesion molecule | | | | | | | S | S | | S | | |
| NF-kappa-B inhibitor alpha | | S | | | S | | | | | | S | S |
| Nidogen-1 | S | | | | | | S | | S | S | | |
| Osteocalcin | S | | | | | | S | | S | S | | S |
| Pappalysin-1 | S | | S | S | S | | S | | S | S | S | S |
| Poly [ADP-ribose] polymerase 1 (cleaved) | S | | | | | | | | | | | |
| Probetacellulin | S | | | | | | | | | | | |
| Prostate-specific antigen | | S | | | | | | | | | | |
| Prostatic acid phosphatase | | | | | S | | | S | | | | |
| Protein NOV homolog | | | | | | | | | | | S | |
| Protransforming growth factor alpha | S | S | | S | | | S | | | | S | S |
| P-selectin glycoprotein ligand 1 | | S | | S | | | S | S | S | S | | S |
| Sex hormone-binding globulin | | | | S | | | | | | | | |
| Tenascin | | | | | | | | S | | | | |
| Thrombospondin-2 | | | | S | | | | S | | | | |
| Thymic stromal lymphopoietin | S | S | | | S | | S | | S | | | |
| Transmembrane glycoprotein NMB | S | | | S | | | | | S | S | | |
| Trefoil factor 3 | S | | S | S | | | | S | | | | |
| Tubulointerstitial nephritis antigen | S | | | | | | | | | | | |
| Tumor necrosis factor receptor superfamily member | S | | S | S | | | S | | | | S | S |
| 10B | | | | | | | | | | | | |
| Tumor necrosis factor receptor superfamily member 8 | S | | | | | | | S | | | | |
| Versican core protein | | | | | | | S | S | S | S | S | |

| | | | | | | | | | | | | |
|---|---|---|---|---|---|---|---|---|---|---|---|---|
| Key for the 12 different uses of the biomarkers in Table 1: **1** = use of analyte in urine as a marker for sepsis **2** = use of analyte in urine as a marker for progression to sepsis **3** = use of analyte in urine as a marker for septic shock **4** = use of analyte in urine as a marker for progression to septic shock **5** = use of analyte in urine as a marker for death with sepsis **6** = use of analyte in urine as a marker for death with septic shock **7** = use of analyte in plasma as a marker for sepsis **8** = use of analyte in plasma as a marker for progression to sepsis **9** = use of analyte in plasma as a marker for septic shock **10** = use of analyte in plasma as a marker for progression to septic shock **11** = use of analyte in plasma as a marker for death with sepsis **12** = use of analyte in plasma as a marker for death with septic shock S = two-tailed *p*-value ≤ 0.10 for AUC | | | | | | | | | | | | |

### Example 9: Sepsis patient sample collection (2)

The objective of this study is to collect samples from acutely ill patients. Approximately 800 adults expected to be in the ICU for at least 48 hours will be enrolled. To be enrolled in the study, each patient must meet all of the following inclusion criteria and none of the following exclusion criteria:
Inclusion Criteria
   males and females 21 years of age or older, within 24 hours of being admitted into the ICU, expected to have an indwelling urinary catheter for at least 48 hours after enrollment, and have at least one of the following acute conditions within 24 hours prior to enrollment:
   (i) respiratory SOFA score of ≥ 2 (PaO2/FiO2 <300),
   (ii) cardiovascular SOFA score of ≥ 1 (MAP < 70 mm Hg and/or any vasopressor required).
Exclusion Criteria
   known pregnancy;
   institutionalized individuals;
   previous renal transplantation;
   known acutely worsening renal function prior to enrollment (e.g., any category of RIFLE criteria);
   received dialysis (either acute or chronic) within 5 days prior to enrollment or in imminent need of dialysis at the time of enrollment;
   known infection with human immunodeficiency virus (HIV) or a hepatitis virus;
   meets any of the following:
   (i) active bleeding with an anticipated need for > 4 units PRBC in a day;
   (ii) hemoglobin < 7 g/dL;
   (iii) any other condition that in the physician's opinion would contraindicate drawing serial blood samples for clinical study purposes;
   meets only the SBP < 90 mmHg inclusion criterion set forth above, and does not have shock in the attending physician's or principal investigator's opinion;

After obtaining informed consent, an EDTA anti-coagulated blood sample (10 mL) and a urine sample (25-50 mL) are collected from each patient. Blood and urine samples are then collected at 12 (± 1), 24 (± 2), 36 (± 2), 48 (± 2), 60 (± 2), 72 (± 2), and 84 (± 2) hours after enrollment, and thereafter daily up to day 7 to day 14 while the subject is hospitalized. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

### Example 10. Use of analyte as a marker for sepsis

Patients from the intensive care unit (ICU) were classified as positive or negative for sepsis according to clinical diagnosis on each day from enrollment to 6 days after.

Two cohorts were defined as (Cohort 1) patients who had sepsis, and (Cohort 2) patients who did not have sepsis on any day from enrollment to 6 days after (7 days total). Both urine and plasma samples from each patient in Cohorts 1 and 2 were collected on the day of enrollment. The concentrations of the analyte in these samples were measured by standard immunoassay methods using commercially available assay reagents. A receiver operating characteristic (ROC) curve was generated using the concentrations, and the performance of the analyte was assessed by the area under the ROC curve (AUC). The two-tailed *p*-value of the AUC for the analyte was calculated, and if the p-value was less than 0.1, the AUC was considered statistically significant.

### Example 11. Use of analyte as a marker for progression to sepsis

Two cohorts were defined as (Cohort 1) patients who did not have sepsis during the 5 days prior to enrollment but had sepsis on any of the subsequent 7 days (enrollment to 6 days after), and (Cohort 2) patients who did not have sepsis on any of the 5 days prior to enrollment and on any of the subsequent 7 days. The urine and plasma sample collections and analyses using the measured analyte concentrations were the same as in Example 3 above.

### Example 12. Use of analyte as a marker for septic shock

Two cohorts were defined as (Cohort 1) patients who had both sepsis and hypotension on the same day (septic shock), and (Cohort 2) patients who did not have septic shock on any day from enrollment to 6 days after. A patient was classified as having hypotension if his/her systolic blood pressure was below 70mm Hg. The urine and plasma sample collections and analyses using the measured analyte concentrations were the same as in Example 3 above.

### Example 13. Use of analyte as a marker for progression to septic shock

Two cohorts were defined as (Cohort 1) patients who did not have septic shock (see Example 5 for definition) on any of the 5 days prior to enrollment but had septic shock on any of the subsequent 7 days (enrollment to 6 days after), and (Cohort 2) patients who did not have septic shock on any of the 5 days prior to enrollment and on any of the subsequent 7 days. The urine and plasma sample collections and analyses using the measured analyte concentrations were the same as in Example 3 above.

### Example 14. Use of analyte as a marker for death with sepsis

Two cohorts were defined as (Cohort 1) patients who had sepsis on any day from enrollment to 6 days after and died within 30 days after enrollment, and (Cohort 2) patients who did not die and patients who died within 30 days after enrollment but did not have sepsis on any day from enrollment to 6 days after. The urine and plasma sample collections and analyses using the measured analyte concentrations were the same as in Example 3 above.

### Example 15. Use of analyte as a marker for death with septic shock

Two cohorts were defined as (Cohort 1) patients who had septic shock (see Example 5 for definition) on any day from enrollment to 6 days after and died within 30 days after enrollment, and (Cohort 2) patients who did not die and patients who died within 30 days after enrollment but did not have septic shock on any day from enrollment to 6 days after. The urine and plasma sample collections and analyses using the measured analyte concentrations were the same as in Example 3 above.

**Table 2 Use of Biomarkers in Sepsis**

| **Preferred Name** | **1** | **2** | **3** | **4** | **5** | **6** | **7** | **8** | **9** | **10** | **11** | **12** |
|---|---|---|---|---|---|---|---|---|---|---|---|---|
| Insulin-like growth factor-binding protein 6 | S | S | S | S | | | | | S | S | | |
| Clusterin | S | S | S | S | | | | | S | S | S | S |
| Tumor necrosis factor receptor superfamily member 10B | S | S | S | S | | | | | | | S | |
| Trefoil factor 3 | S | S | S | S | | | | | | | | |
| Thrombospondin-2 | S | S | S | S | | | | | | | | |
| Cathepsin S | S | S | S | S | | | | | | | | |
| Hepatitis A virus cellular receptor 1 | S | S | S | S | | | | | | | S | S |
| Carbonic anhydrase 9 | S | S | S | S | | | | | | | | |
| Protein NOV homolog | S | S | S | S | | | | | | | | |
| Interleukin-29 | S | S | S | S | | | | | | | S | S |
| C-X-C motif chemokine 11 | S | S | S | S | | | | | | | | |
| Insulin-like growth factor-binding protein 7 | S | S | S | S | | | | | | | S | |
| C-X-C motif chemokine 16 | S | S | S | S | | | | | S | | S | S |
| C-C motif chemokine 15 | S | S | S | S | | | | | | | | |
| Galectin-3 | S | S | S | S | | | | | | | | |
| Transmembrane glycoprotein NMB | S | S | S | S | | | | | | | S | |
| C-C motif chemokine 18 | S | S | S | S | | | | | | | S | |
| Angiopoietin-related protein 4 | S | S | S | S | | | | | | | S | |
| Glucagon | S | S | S | | | | | | | | | |
| Cancer Antigen 15-3 | S | S | S | S | | | | | | | | |
| Endostatin | S | | S | S | | | | | | | | |
| Angiopoietin-related protein 6 | S | S | S | S | | | | S | | | | |
| Epidermal growth factor receptor | S | S | S | S | | | | | | | | |
| Nidogen-1 | S | S | S | S | | | | | S | | | |
| Apolipoprotein(a) | | | | S | | | | | S | S | S | S |
| Carcinoembryonic antigen-related cell adhesion molecule 1 | S | S | S | S | | | | | | | S | S |
| Angiopoietin-1 receptor | S | | S | S | | | | | | | S | S |
| Poly [ADP-ribose] polymerase 1 (cleaved) | S | | S | | | | | | | | | |
| Lymphatic vessel endothelial hyaluronic acid receptor 1 | S | S | S | S | | | | | | | S | |
| Tumor necrosis factor receptor superfamily member 8 | S | | S | S | | | | | | | | |
| Collagenase 3 | S | | S | S | | | | | | | | |
| C-C motif chemokine 20 | S | | S | | | | | | S | | S | S |
| Carcinoembryonic antigen-related cell adhesion molecule 5 | S | | S | S | | | | | | | | |
| Glucagon-like peptide 1 | S | S | S | | | | | | | | | |
| C-C motif chemokine 17 | S | S | S | S | | | | | | | | |
| NF-kappa-B inhibitor alpha | S | | S | S | | | | | | | | |
| Vascular endothelial growth factor D | | | | | | | | | S | | | |
| Metalloproteinase inhibitor 3 | S | | S | S | | | | | | | | |
| Osteocalcin | | S | | | | | | S | S | | | S |
| Thymic stromal lymphopoietin | S | S | S | S | | | | | S | | | |
| Vascular endothelial growth factor receptor 2 | S | | S | S | | | | | | | | |
| Involucrin | S | | S | S | | | | | S | | S | |
| Protransforming growth factor alpha | S | S | S | S | | | | | | | S | |
| P-selectin glycoprotein ligand 1 | S | S | S | S | | | | | | | | |
| Insulin | S | | | | | | | | | | S | |
| Neuronal cell adhesion molecule | | | | S | | | S | | | | | S |
| Apolipoprotein C-III | S | | S | S | | | | | | | S | |
| SL cytokine | S | S | | | | | | | | | | |
| Heparin-binding growth factor 1 | | | | | | | S | | | | S | S |
| Angiogenin | | | | | | | S | | | | S | S |
| Epiregulin | S | | S | S | | | | | | | | |
| Bone morphogenetic protein 7 | S | S | S | S | | | | | | | | |
| Glutathione S-transferase P | | | | | | | S | | | | S | |
| Cancer Antigen 19-9 | | | | | | | S | | | | | |
| Apolipoprotein A-II | | | | | | | S | | | | S | |
| Epithelial cell adhesion molecule | S | | | | | | | | | | | S |
| Cadherin-3 | | S | | S | | | S | | | | S | S |
| Tenascin | | | | | | | S | | | | S | |
| C-Peptide | | | S | S | | | | | | | | |
| C-C motif chemokine 21 | | | S | S | | | | | | | | |
| Angiopoietin-related protein 3 | | | S | S | | | | | | | | |
| Interleukin-21 | | | | | | | | | | | S | |
| Myeloid differentiation primary response protein MyD88 | | | | | | | | | | | | S |
| Vascular endothelial growth factor receptor 3 | | | | S | | | | | | | | |
| Islet amyloid polypeptide | | | | S | | | | | | | | |
| Insulin-like growth factor 1 receptor | | | | | S | S | | | | | | |
| Macrophage metalloelastase | | | | | S | | | | | | | |
| Fibroblast growth factor 19 | | | | | S | | | | | | | |

| | | | | | | | | | | | | |
|---|---|---|---|---|---|---|---|---|---|---|---|---|
| Key for the 12 different uses of the biomarkers in Table 1: **1** = use of analyte in urine as a marker for sepsis **2** = use of analyte in urine as a marker for progression to sepsis **3** = use of analyte in urine as a marker for septic shock **4** = use of analyte in urine as a marker for progression to septic shock **5** = use of analyte in urine as a marker for death with sepsis **6** = use of analyte in urine as a marker for death with septic shock **7 =** use of analyte in plasma as a marker for sepsis **8 =** use of analyte in plasma as a marker for progression to sepsis **9 =** use of analyte in plasma as a marker for septic shock **10** = use of analyte in plasma as a marker for progression to septic shock **11** = use of analyte in plasma as a marker for death with sepsis **12** = use of analyte in plasma as a marker for death with septic shock S = two-tailed *p*-value ≤ 0.10 for AUC | | | | | | | | | | | | |

The examples provided herein are representative of preferred embodiments and are exemplary.

Thus, for example, in each instance herein any of the terms "comprising", "consisting essentially of' and "consisting of' may be replaced with either of the other two terms.

## Claims

1. An in vitro method of diagnosing SIRS, sepsis, severe sepsis, septic shock, or multiple organ dysfunction syndrome (MODS) in a subject, or assigning a prognostic risk for one or more clinical outcomes for a subject suffering from SIRS, sepsis, severe sepsis, septic shock, or MODS, the method comprising:
performing one or more assays configured to detect Insulin-like growth factor-binding protein 7, and optionally further one or more biomarkers selected from the group consisting of Alpha-2 macroglobulin, Angiogenin, Angiopoietin-1 receptor, Angiopoietin-related protein 3, Angiopoietin-related protein 4, Angiopoietin-related protein 6, Apolipoprotein A-II, Apolipoprotein C-III, Apolipoprotein(a), Bone morphogenetic protein 7, Cadherin-16, Cadherin-3, Cancer Antigen 15-3, Cancer Antigen 19-9, Carbonic anhydrase 9, Carcinoembryonic antigen-related cell adhesion molecule 1, Carcinoembryonic antigen-related cell adhesion molecule 5, Caspase-3, active, Cathepsin S, C-C motif chemokine 1, C-C motif chemokine 15, C-C motif chemokine 17 , C-C motif chemokine 18, C-C motif chemokine 20, C-C motif chemokine 21, C-C motif chemokine 24, Clusterin, Collagenase 3, C-peptide (Insulin), C-X-C motif chemokine 11, C-X-C motif chemokine 16, Cyclin-dependent kinase inhibitor 1, DDRGK domain-containing protein 1, Endostatin, Epidermal growth factor receptor, Epiregulin, Epithelial cell adhesion molecule, Erythropoietin receptor, Fibroblast growth factor 19, Fibroblast growth factor 23, Galectin-3, Gastric inhibitory polypeptide, Glucagon, Glucagon-like peptide 1 , Glutathione S-transferase P, Heat shock protein beta-1, Heparin-binding growth factor 1, Hepatitis A virus cellular receptor 1, Hepatocyte growth factor receptor, Insulin, Insulin-like growth factor 1 receptor, Insulin-like growth factor-binding protein 6, Interferon alpha-2, Interleukin-20, Interleukin-21, Interleukin-28A, Interleukin-29, Interleukin-33, Involucrin, Islet amyloid polypeptide, Keratin, type II cytoskeletal 1 (Keratin-1,-10 mix), Lymphatic vessel endothelial hyaluronic acid receptor 1, Macrophage metalloelastase, Matrix metalloproteinase-9:Metalloproteinase inhibitor 2 complex, Metalloproteinase inhibitor 3, Myeloid differentiation primary response protein MyD88, Netrin-1, Netrin-4, Neuronal cell adhesion molecule, NF-kappa-B inhibitor alpha, Nidogen-1, Osteocalcin, Pappalysin-1, Poly [ADP-ribose] polymerase 1 (cleaved), Probetacellulin, Prostate-specific antigen, Prostatic acid phosphatase, Protein NOV homolog, Protransforming growth factor alpha, P-selectin glycoprotein ligand 1, Sex hormone-binding globulin, SL cytokine, Tenascin, Thrombospondin-2, Thymic stromal lymphopoietin , Transmembrane glycoprotein NMB, Trefoil factor 3, Tubulointerstitial nephritis antigen, Tumor necrosis factor receptor superfamily member 10B, Tumor necrosis factor receptor superfamily member 8, Versican core protein, Bone morphogenetic protein 7, Carbonic anhydrase 9, Caspase-9, Collagenase 3, Granzyme M, Heparin-binding EGF-like growth factor, Insulin receptor substrate 1, Keratin, type II cytoskeletal 6 (6A, -6B, -6C mix), Myeloid differentiation primary response protein MyD88, SL cytokine, Vascular endothelial growth factor D, Vascular endothelial growth factor receptor 2, and Vascular endothelial growth factor receptor 3 on a body fluid sample obtained from the subject for the method of diagnosing or on a urine sample for the method of assessing the prognostic risk to provide one or more assay result(s); and
comparing the assay result(s) in the form of a Insulin-like growth factor-binding protein 7 concentration to a predetermined threshold selected to be indicative of the presence or absence of SIRS, the presence or absence of sepsis, the presence or absence of severe sepsis, the presence or absence of septic shock, the presence or absence of MODS, or comparing the assay result(s) in the form of a Insulin-like growth factor-binding protein 7 concentration to a predetermined threshold selected to be indicative of the prognostic risk of one or more clinical outcomes, wherein the one or more clinical outcome comprises a prognostic risk of mortality for the subject suffering from or believed to suffer from SIRS, sepsis, severe sepsis, septic shock, or MODS.

2. A method according to claim 1, wherein said relating step comprises comparing each of the immunoassay result(s) to a corresponding predetermined threshold level selected to provide a sensitivity or specificity of at least 0.7 for the diagnosis of sepsis, compared to SIRS not progressed to sepsis.

3. A method according to claim 1, wherein said relating step comprises comparing each of the immunoassay result(s) to a corresponding predetermined threshold level selected to provide a sensitivity or specificity of at least 0.7 for the diagnosis of severe sepsis, compared to SIRS not progressed to severe sepsis.

4. A method according to claim 1, wherein said relating step comprises comparing each of the immunoassay result(s) to a corresponding predetermined threshold level selected to provide a sensitivity or specificity of at least 0.7 for the diagnosis of septic shock, compared to SIRS not progressed to septic shock.

5. A method according to claim 1, wherein said relating step comprises comparing each of the immunoassay result(s) to a corresponding predetermined threshold level selected to provide an odds ratio of at least 2 for the prognostic risk of mortality.

6. A method according to claim 1, wherein said relating step comprises comparing each of the immunoassay result(s) to a corresponding predetermined threshold level selected to provide an odds ratio of at least 2 for the prognostic risk of a worsening from sepsis to severe sepsis; from sepsis or severe sepsis to septic shock; from sepsis, severe sepsis, or septic shock to MODS.

7. A method according to claim 1, wherein the body fluid sample is selected from the group consisting of blood, serum, and plasma, orwherein the method is a prognostic method, and the method differentiates between a risk of future sepsis and a risk of future severe sepsis or septic shock.

8. A method according to claim 1, wherein the method is a prognostic method, and the method differentiates between a risk of future sepsis or severe sepsis and a risk of future septic shock or wherein the method is a diagnostic method, and the method differentiates between a diagnosis of sepsis and a diagnosis of severe sepsis or septic shock, or
wherein the method is a diagnostic method, and the method differentiates between a diagnosis of sepsis or severe sepsis and a diagnosis of septic shock.

9. An in vitro method for evaluating biomarker levels in a body fluid sample, comprising:
performing, on a body fluid sample obtained from a subject selected for evaluation based on a determination that the subject is at risk of a future or current diagnosis of sepsis, severe sepsis, septic shock or MODS, one or more analyte binding assays configured to detect Insulin-like growth factor-binding protein 7, and optionally further one or more biomarkers selected from the group consisting of Alpha-2 macroglobulin, Angiogenin, Angiopoietin-1 receptor, Angiopoietin-related protein 3, Angiopoietin-related protein 4, Angiopoietin-related protein 6, Apolipoprotein A-II, Apolipoprotein C-III, Apolipoprotein(a), Bone morphogenetic protein 7, Cadherin-16, Cadherin-3, Cancer Antigen 15-3, Cancer Antigen 19-9, Carbonic anhydrase 9, Carcinoembryonic antigen-related cell adhesion molecule 1, Carcinoembryonic antigen-related cell adhesion molecule 5, Caspase-3, active, Cathepsin S, C-C motif chemokine 1, C-C motif chemokine 15, C-C motif chemokine 17 , C-C motif chemokine 18, C-C motif chemokine 20, C-C motif chemokine 21, C-C motif chemokine 24, Clusterin, Collagenase 3, C-peptide (Insulin), C-X-C motif chemokine 11, C-X-C motif chemokine 16, Cyclin-dependent kinase inhibitor 1, DDRGK domain-containing protein 1, Endostatin, Epidermal growth factor receptor, Epiregulin, Epithelial cell adhesion molecule, Erythropoietin receptor, Fibroblast growth factor 19, Fibroblast growth factor 23, Galectin-3, Gastric inhibitory polypeptide, Glucagon, Glucagon-like peptide 1 , Glutathione S-transferase P, Heat shock protein beta-1, Heparin-binding growth factor 1, Hepatitis A virus cellular receptor 1, Hepatocyte growth factor receptor, Insulin, Insulin-like growth factor 1 receptor, Insulin-like growth factor-binding protein 6, Interferon alpha-2, Interleukin-20, Interleukin-21, Interleukin-28A, Interleukin-29, Interleukin-33, Involucrin, Islet amyloid polypeptide, Keratin, type II cytoskeletal 1 (Keratin-1,-10 mix), Lymphatic vessel endothelial hyaluronic acid receptor 1, Macrophage metalloelastase, Matrix metalloproteinase-9:Metalloproteinase inhibitor 2 complex, Metalloproteinase inhibitor 3, Myeloid differentiation primary response protein MyD88, Netrin-1, Netrin-4, Neuronal cell adhesion molecule, NF-kappa-B inhibitor alpha, Nidogen-1, Osteocalcin, Pappalysin-1, Poly [ADP-ribose] polymerase 1 (cleaved), Probetacellulin, Prostate-specific antigen, Prostatic acid phosphatase, Protein NOV homolog, Protransforming growth factor alpha, P-selectin glycoprotein ligand 1, Sex hormone-binding globulin, SL cytokine, Tenascin, Thrombospondin-2, Thymic stromal lymphopoietin , Transmembrane glycoprotein NMB, Trefoil factor 3, Tubulointerstitial nephritis antigen, Tumor necrosis factor receptor superfamily member 10B, Tumor necrosis factor receptor superfamily member 8, Versican core protein, Bone morphogenetic protein 7, Carbonic anhydrase 9, Caspase-9, Collagenase 3, Granzyme M, Heparin-binding EGF-like growth factor, Insulin receptor substrate 1, Keratin, type II cytoskeletal 6 (6A, -6B, -6C mix), Myeloid differentiation primary response protein MyD88, SL cytokine, Vascular endothelial growth factor D, Vascular endothelial growth factor receptor 2, and Vascular endothelial growth factor receptor 3 by introducing the body fluid sample obtained from the subject into an assay instrument which (i) contacts a plurality of reagents which specifically bind for detection the plurality of biomarkers with the urine sample, and (ii) generates one or more assay results indicative of binding of each biomarker which is assayed to a respective specific binding reagent in the plurality of reagents;
comparing the assay result(s) in the form of a Insulin-like growth factor-binding protein 7 concentration to a predetermined threshold selected to be indicative of a risk of a future or current diagnosis of sepsis, severe sepsis, septic shock or MODS, and
displaying the one or more assay results from the assay instrument as a quantitative result in a human-readable form.

10. A method according to claim 9, wherein the subject is selected for evaluation based on a determination that the subject has sepsis and is at risk of a future diagnosis of severe sepsis, septic shock or MODS, or
wherein the subject is selected for evaluation based on a determination that the subject has sepsis or severe sepsis and is at risk of a future diagnosis of septic shock or MODS, or
wherein a plurality of assay results are combined using a function that converts said assay results into a single composite result, or
wherein the subject is selected for evaluation based on a determination that the subject is at risk of a future diagnosis of severe sepsis, septic shock or MODS within a period selected from the group consisting of 21 days, 14 days, 7 days, 5 days, 96 hours, 72 hours, 48 hours, 36 hours, 24 hours, and 12 hours, or
wherein the subject is selected for evaluation based on a determination that the subject is at risk of a future diagnosis of one or more of an injury to renal function, reduced renal function, improvement in renal function, and future ARF, or
wherein the plurality of assays are immunoassays performed by (i) introducing the urine sample into an assay device comprising a plurality of antibodies, at least one of which binds to each biomarker which is assayed, and (ii) generating an assay result indicative of binding of each biomarker to its respective antibody .

## Patentansprüche

1. In-vitro-Verfahren zur Diagnose von SIRS, Sepsis, schwerer Sepsis, septischem Schock oder Multiorgandysfunktionssyndrom (MODS) bei einem Subjekt oder zum Zuordnen eines prognostischen Risikos für ein oder mehrere klinische Resultate für ein Subjekt, das an SIRS, Sepsis, schwerer Sepsis, septischem Schock oder MODS leidet, wobei das Verfahren Folgendes beinhaltet:
Durchführen von einem oder mehreren Assays, die so konfiguriert sind, dass sie insulinähnliches Wachstumsfaktor-Bindungsprotein 7 und optional ferner einen oder mehrere Biomarker detektieren, ausgewählt aus der Gruppe bestehend aus Alpha-2-Makroglobulin, Angiogenin, Angiopoietin-1-Rezeptor, Angiopoietin-verwandtem Protein 3, Angiopoietin-verwandtem Protein 4, Angiopoietin-verwandtem Protein 6, Apolipoprotein A-II, Apolipoprotein C-III, Apolipoprotein(a), knochenmorphogenetischem Protein 7, Cadherin-16, Cadherin-3, Krebsantigen 15-3, Krebsantigen 19-9, Carboanhydrase 9, carcinoembryonales-Antigen-verwandtem Zelladhäsionsmolekül 1, carcinoembryonales-Antigen-verwandtem Zelladhäsionsmolekül 5, Caspase-3, aktiv, Cathepsin S, C-C-Motiv-Chemokin 1, C-C-Motiv-Chemokin 15, C-C-Motiv-Chemokin 17, C-C-Motiv-Chemokin 18, C-C-Motiv-Chemokin 20, C-C-Motiv-Chemokin 21, C-C-Motiv-Chemokin 24, Clusterin, Collagenase 3, C-Peptid (Insulin), C-X-C-Motiv-Chemokin 11, C-X-C-Motiv-Chemokin 16, Cyclinabhängigem Kinase-Inhibitor 1, DDRGK-Domänen-haltigem Protein 1, Endostatin, epidermalem Wachstumsfaktorrezeptor, Epiregulin, Epithelzelladhäsionsmolekül, Erythropoietin-Rezeptor, Fibroblasten-Wachstumsfaktor 19, Fibroblasten-Wachstumsfaktor 23, Galectin-3, gastroinhibitorischem Polypeptid, Glucagon, Glucagon-ähnlichem Peptid 1, Glutathion-S-Transferase P, Hitzeschockprotein Beta-1, heparinbindendem Wachstumsfaktor 1, Hepatitis-A-Virus-Zellrezeptor 1, Hepatozyten-Wachstumsfaktor-Rezeptor, Insulin, insulinähnlichem Wachstumsfaktor-1-Rezeptor, insulinähnlichem Wachstumsfaktor-Bindungsprotein 6, Interferon Alpha-2, Interleukin-20, Interleukin-21, Interleukin-28A, Interleukin-29, Interleukin-33, Involucrin, Insel-Amyloid-Polypeptid, Keratin, Typ II Zytoskelett 1 (Keratin-1,-10 Mischung), endothelialem Hyaluronsäurerezeptor 1 der Lymphgefäße, Makrophagen-Metalloelastase, Matrix-Metalloproteinase-9:Metalloproteinase-Inhibitor-2-Komplex, Metalloproteinase-Inhibitor-3, MyD88 (Myeloid Differentiation Primary Response Protein), Netrin-1, Netrin-4, neuronalem Zelladhäsionsmolekül, NF-Kappa-B-Inhibitor Alpha, Nidogen-1, Osteocalcin, Pappalysin-1, Poly[ADP-Ribose]Polymerase 1 (gespalten), Probetacellulin, Prostata-spezifischem Antigen, prostataspezifischer saurer Phosphatase, Protein NOV-Homolog, protransformierendem Wachstumsfaktor Alpha, P-Selektin-Glykoprotein-Ligand 1, Sexualhormon-bindendem Globulin, SL-Zytokin, Tenascin, Thrombospondin-2, thymischem stromalem Lymphopoietin, Transmembranglykoprotein NMB, Trefoil-Faktor 3, tubulointerstitielle-Nephritis-Antigen, Tumornekrosefaktorrezeptor-Superfamilienmitglied 10B, Tumornekrosefaktorrezeptor-Superfamilienmitglied 8, Versican-Kernprotein, knochenmorphogenetischem Protein 7, Carboanhydrase 9, Caspase-9, Collagenase 3, Granzym M, heparinbindendem EGF-ähnlichem Wachstumsfaktor, Insulinrezeptorsubstrat 1, Keratin, Typ II Zytoskelett 6 (6A, -6B, -6C Mischung), MyD88 (Myeloid Differentiation Primary Response Protein), SL-Zytokin, vaskulärem endothelialem Wachstumsfaktor D, vaskulärem endothelialem Wachstumsfaktorrezptor 2 und vaskulärem endothelialem Wachstumsfaktorrezeptor 3, an einer Körperflüssigkeitsprobe, die von dem Subjekt für das Diagnoseverfahren erhalten wurde, oder an einer Urinprobe für das Verfahren zur Bewertung des prognostischen Risikos, um ein oder mehrere Assay-Ergebnisse zu erhalten; und
Vergleichen des/der Assay-Ergebnisse(s) in Form einer insulinähnlichen Wachstumsfaktor-Bindungsprotein-7-Konzentration mit einem vorbestimmten Schwellenwert, der so gewählt wird, dass er auf die An- oder Abwesenheit von SIRS, die An- oder Abwesenheit von Sepsis, die An- oder Abwesenheit von schwerer Sepsis, die An- oder Abwesenheit von septischem Schock, die An- oder Abwesenheit von MODS hindeutet, oder Vergleichen des/der Assay-Ergebnisse(s) in Form einer insulinähnlichen Wachstumsfaktor-Bindungsprotein-7-Konzentration mit einem vorbestimmten Schwellenwert, der so gewählt wird, dass er auf das prognostische Risiko für ein oder mehrere klinische Resultate hindeutet, wobei die ein oder mehreren klinischen Resultate ein prognostisches Mortalitätsrisiko für das Subjekt beinhalten, das an SIRS, Sepsis, schwerer Sepsis, septischem Schock oder MODS leidet oder vermutlich leidet.

2. Verfahren nach Anspruch 1, wobei der genannte Schritt des Inbeziehungbringens das Vergleichen jedes der Immunoassay-Ergebnisse mit einem entsprechenden vorbestimmten Schwellenwert beinhaltet, der so gewählt wird, dass er eine Empfindlichkeit oder Spezifität von wenigstens 0,7 für die Diagnose einer Sepsis im Vergleich zu SIRS, das sich nicht zu einer Sepsis entwickelt, bereitstellt.

3. Verfahren nach Anspruch 1, wobei der genannte Schritt des Inbeziehungbringens das Vergleichen jedes der Immunoassay-Ergebnisse mit einem entsprechenden vorbestimmten Schwellenwert beinhaltet, der so gewählt wird, dass eine Empfindlichkeit oder Spezifität von wenigstens 0,7 für die Diagnose einer schweren Sepsis im Vergleich zu SIRS, das sich nicht zu einer schweren Sepsis entwickelt, bereitgestellt wird.

4. Verfahren nach Anspruch 1, wobei der genannte Schritt des Inbeziehungbringens das Vergleichen jedes der Immunoassay-Ergebnisse mit einem entsprechenden vorbestimmten Schwellenwert beinhaltet, der so gewählt wird, dass eine Empfindlichkeit oder Spezifität von wenigstens 0,7 für die Diagnose eines septischen Schocks im Vergleich zu SIRS, das sich nicht zu einem septischen Schock entwickelt, bereitgestellt wird.

5. Verfahren nach Anspruch 1, wobei der genannte Schritt des Inbeziehungbringens das Vergleichen jedes der Immunoassay-Ergebnisse mit einem entsprechenden vorbestimmten Schwellenwert beinhaltet, der so gewählt wird, dass ein Chancenverhältnis von wenigstens 2 für das prognostische Mortalitätsrisiko bereitgestellt wird.

6. Verfahren nach Anspruch 1, wobei der genannte Schritt des Inbeziehungbringens das Vergleichen jedes der Immunoassay-Ergebnisse mit einem entsprechenden vorbestimmten Schwellenwert beinhaltet, der so gewählt wird, dass ein Chancenverhältnis von wenigstens 2 für das prognostische Risiko einer Verschlechterung einer Sepsis zu einer schweren Sepsis; einer Sepsis oder schweren Sepsis zu einem septischen Schock; einer Sepsis, schweren Sepsis oder eines septischen Schocks zu MODS bereitgestellt wird.

7. Verfahren nach Anspruch 1, wobei die Körperflüssigkeitsprobe ausgewählt wird aus der Gruppe bestehend aus Blut, Serum und Plasma, oder wobei das Verfahren ein Prognoseverfahren ist und das Verfahren zwischen einem Risiko für eine zukünftige Sepsis und einem Risiko für eine(n) zukünftige(n) schwere Sepsis oder septischen Schock differenziert.

8. Verfahren nach Anspruch 1, wobei das Verfahren ein Prognoseverfahren ist und das Verfahren zwischen einem Risiko für eine zukünftige Sepsis oder schwere Sepsis und einem Risiko für einen zukünftigen septischen Schock differenziert oder wobei das Verfahren ein Diagnoseverfahren ist und das Verfahren zwischen einer Diagnose einer Sepsis und einer Diagnose einer schweren Sepsis oder eines septischen Schocks differenziert, oder
wobei das Verfahren ein Diagnoseverfahren ist und das Verfahren zwischen einer Diagnose einer Sepsis oder schweren Sepsis und einer Diagnose eines septischen Schocks differenziert.

9. In-vitro-Verfahren zum Beurteilen von Biomarker-Niveaus in einer Körperflüssigkeitsprobe, das Folgendes beinhaltet:
Durchführen, an einer Körperflüssigkeitsprobe, die von einem Subjekt erhalten wurde, das aufgrund einer Feststellung, dass das Subjekt für eine zukünftige oder aktuelle Diagnose von Sepsis, schwerer Sepsis, septischem Schock oder MODS gefährdet ist, für eine Beurteilung ausgewählt wurde, von einem oder mehreren Analytenbindungsassays, die so konfiguriert sind, dass sie insulinähnliches Wachstumsfaktor-Bindungsprotein 7 und optional ferner einen oder mehrere Biomarker detektieren, ausgewählt aus der Gruppe bestehend aus Alpha-2-Makroglobulin, Angiogenin, Angiopoietin-1-Rezeptor, Angiopoietin-verwandtem Protein 3, Angiopoietin-verwandtem Protein 4, Angiopoietin-verwandtem Protein 6, Apolipoprotein A-II, Apolipoprotein C-III, Apolipoprotein(a), knochenmorphogenetischem Protein 7, Cadherin-16, Cadherin-3, Krebsantigen 15-3, Krebsantigen 19-9, Carboanhydrase 9, carcinoembryonales-Antigen-verwandtem Zelladhäsionsmolekül 1, carcinoembryonales- Antigen-verwandtem Zelladhäsionsmolekül 5, Caspase-3, aktiv, Cathepsin S, C-C-Motiv-Chemokin 1, C-C-Motiv-Chemokin 15, C-C-Motiv-Chemokin 17, C-C-Motiv-Chemokin 18, C-C-Motiv-Chemokin 20, C-C-Motiv-Chemokin 21, C-C-Motiv-Chemokin 24, Clusterin, Collagenase 3, C-Peptid (Insulin), C-X-C-Motiv-Chemokin 11, C-X-C-Motiv-Chemokin 16, Cyclinabhängigem Kinase-Inhibitor 1, DDRGK-Domänen-haltigem Protein 1, Endostatin, epidermalem Wachstumsfaktorrezeptor, Epiregulin, Epithelzelladhäsionsmolekül, Erythropoietin-Rezeptor, Fibroblasten-Wachstumsfaktor 19, Fibroblasten-Wachstumsfaktor 23, Galectin-3, gastroinhibitorischem Polypeptid, Glucagon, Glucagon-ähnlichem Peptid 1, Glutathion-S-Transferase P, Hitzeschockprotein Beta-1, heparinbindendem Wachstumsfaktor 1, Hepatitis-A-Virus-Zellrezeptor 1, Hepatozyten-Wachstumsfaktor-Rezeptor, Insulin, insulinähnlichem Wachstumsfaktor-1-Rezeptor, insulinähnlichem Wachstumsfaktor-Bindungsprotein 6, Interferon Alpha-2, Interleukin-20, Interleukin-21, Interleukin-28A, Interleukin-29, Interleukin-33, Involucrin, Insel-Amyloid-Polypeptid, Keratin, Typ II Zytoskelett 1 (Keratin-1,-10 Mischung), endothelialem Hyaluronsäurerezeptor 1 der Lymphgefäße, Makrophagen-Metalloelastase, Matrix-Metalloproteinase-9:Metalloproteinase-Inhibitor-2-Komplex, Metalloproteinase-Inhibitor-3, MyD88 (Myeloid Differentiation Primary Response Protein), Netrin-1, Netrin-4, neuronalem Zelladhäsionsmolekül, NF-Kappa-B-Inhibitor Alpha, Nidogen-1, Osteocalcin, Pappalysin-1, Poly[ADP-Ribose]Polymerase 1 (gespalten), Probetacellulin, Prostata-spezifischem Antigen, prostataspezifischer saurer Phosphatase, Protein NOV-Homolog, protransformierendem Wachstumsfaktor Alpha, P-Selektin-Glykoprotein-Ligand 1, Sexualhormon-bindendem Globulin, SL-Zytokin, Tenascin, Thrombospondin-2, thymischem stromalem Lymphopoietin, Transmembranglykoprotein NMB, Trefoil-Faktor 3, tubulointerstitielle-Nephritis-Antigen, Tumornekrosefaktorrezeptor-Superfamilienmitglied 10B, Tumornekrosefaktorrezeptor-Superfamilienmitglied 8, Versican-Kernprotein, knochenmorphogenetischem Protein 7, Carboanhydrase 9, Caspase-9, Collagenase 3, Granzym M, heparinbindendem EGF-ähnlichem Wachstumsfaktor, Insulinrezeptorsubstrat 1, Keratin, Typ II Zytoskelett 6 (6A, -6B, -6C Mischung), MyD88 (Myeloid Differentiation Primary Response Protein), SL-Zytokin, vaskulärem endothelialem Wachstumsfaktor D, vaskulärem endothelialem Wachstumsfaktorrezptor 2 und vaskulärem endothelialem Wachstumsfaktorrezeptor 3, durch Einbringen der von dem Subjekt erhaltenen Körperflüssigkeitsprobe in ein Assay-Instrument, das (i) eine Mehrzahl von Reagenzien, die zur Detektion die Mehrzahl von Biomarkern spezifisch binden, mit der Urinprobe in Kontakt bringt, und (ii) ein oder mehrere Assay-Ergebnisse erzeugt, die auf eine Bindung von jedem untersuchten Biomarker an ein entsprechendes spezifisches Bindungsreagenz in der Mehrzahl von Reagenzien hindeutet;
Vergleichen des/der Assay-Ergebnisse(s) in Form einer insulinähnlichen Wachstumsfaktor-Bindungsprotein-7-Konzentration mit einem vorgegebenen Schwellenwert, der so gewählt wird, dass er auf ein Risiko für eine zukünftige oder aktuelle Diagnose von Sepsis, schwerer Sepsis, septischem Schock oder MODS hindeutet, und
Anzeigen der ein oder mehreren Assay-Ergebnisse von dem Assay-Instrument als quantitatives Ergebnis in einer menschenlesbaren Form.

10. Verfahren nach Anspruch 9, wobei das Subjekt aufgrund einer Feststellung, dass das Subjekt eine Sepsis hat und für eine zukünftige Diagnose einer schweren Sepsis, eines septischen Schocks oder MODS gefährdet ist, für eine Beurteilung ausgewählt wird, oder
wobei das Subjekt aufgrund einer Feststellung, dass das Subjekt eine Sepsis oder eine schwere Sepsis hat und für eine zukünftige Diagnose eines septischen Schocks oder MODS gefährdet ist, für eine Beurteilung ausgewählt wird, oder
wobei mehrere Assay-Ergebnisse unter Anwendung einer Funktion kombiniert werden, die die genannten Assay-Ergebnisse in ein einziges zusammengesetztes Ergebnis umwandelt, oder
wobei das Subjekt aufgrund einer Feststellung, dass das Subjekt für eine zukünftige Diagnose von schwerer Sepsis, septischem Schock oder MODS innerhalb eines Zeitraums gefährdet ist, der aus der Gruppe bestehend aus 21 Tagen, 14 Tagen, 7 Tagen, 5 Tagen, 96 Stunden, 72 Stunden, 48 Stunden, 36 Stunden, 24 Stunden und 12 Stunden ausgewählt ist, für eine Beurteilung ausgewählt wird, oder
wobei das Subjekt aufgrund einer Feststellung, dass das Subjekt für eine zukünftige Diagnose von einem oder mehreren aus einer Verletzung der Nierenfunktion, reduzierter Nierenfunktion, Verbesserung der Nierenfunktion und zukünftigem ARF gefährdet ist, für eine Beurteilung ausgewählt wird, oder
wobei die Mehrzahl von Assays Immunoassays sind, die durchgeführt werden durch (i) Einbringen der Urinprobe in eine Assay-Vorrichtung, die eine Mehrzahl von Antikörpern umfasst, von denen sich wenigstens einer an die jeweiligen untersuchten Biomarker bindet, und (ii) Erzeugen eines Assay-Ergebnisses, das auf eine Bindung der jeweiligen Biomarker an ihren entsprechenden Antikörper hindeutet.

## Revendications

1. Procédé in vitro de diagnostic d'un syndrome de réponse inflammatoire systémique (SRIS), d'un sepsis, d'un sepsis sévère, d'un choc septique ou d'un syndrome de défaillance multiviscérale (SDMV) chez un sujet, ou d'attribution d'un risque pronostique d'une ou plusieurs issues cliniques pour un sujet souffrant d'un SRIS, d'un sepsis, d'un sepsis sévère, d'un choc septique ou d'un SDMV, le procédé comprenant :
la réalisation d'une ou plusieurs analyses conçues pour détecter la protéine 7 de liaison au facteur de croissance insuline-like, et optionnellement en outre un ou plusieurs biomarqueurs sélectionnés dans le groupe constitué de la macroglobuline alpha-2, de l'angiogénine, du récepteur de l'angiopoïétine-1, de la protéine 3 apparentée à l'angiopoïétine, de la protéine 4 apparentée à l'angiopoïétine, de la protéine 6 apparentée à l'angiopoïétine, de l'apolipoprotéine A-II, de l'apolipoprotéine C-III, de l'apolipoprotéine(a), de la protéine morphogénétique osseuse de type 7, de la cadhérine-16, de la cadhérine-3, de l'antigène de cancer 15-3, de l'antigène de cancer 19-9, de l'anhydrase carbonique 9, de la molécule d'adhésion cellulaire 1 liée à l'antigène carcino-embryonnaire, de la molécule d'adhésion cellulaire 5 liée à l'antigène carcino-embryonnaire, de la caspase-3, active, de la cathepsine S, de la chimiokine 1 à motif C-C, de la chimiokine 15 à motif C-C, de la chimiokine 17 à motif C-C, de la chimiokine 18 à motif C-C, de la chimiokine 20 à motif C-C, de la chimiokine 21 à motif C-C, de la chimiokine 24 à motif C-C, de la clustérine, de la collagénase 3, du peptide C (insuline), de la chimiokine 11 à motif C-X-C, de la chimiokine 16 à motif C-X-C, de l'inhibiteur de kinase cycline-dépendante 1, de la protéine 1 contenant le domaine DDRGK, de l'endostatine, du récepteur du facteur de croissance épidermique, de l'épiréguline, de la molécule d'adhérence des cellules épithéliales, du récepteur de l'érythropoïétine, du facteur de croissance fibroblastique de type 19, du facteur de croissance fibroblastique de type 23, de la galectine 3, du polypeptide inhibiteur gastrique, du glucagon, du glucagon-like peptide 1, de la glutathion S-transférase P, de la protéine du choc thermique bêta-1, du facteur de croissance se liant à l'héparine de type 1, du récepteur cellulaire 1 du virus de l'hépatite A, du récepteur du facteur de croissance des hépatocytes, de l'insuline, du récepteur du facteur de croissance 1 insuline-like, de la protéine 6 de liaison au facteur de croissance insuline-like, de l'interféron alpha-2, de l'interleukine-20, de l'interleukine-21, de l'interleukine-28A, de l'interleukine-29, de l'interleukine-33, de l'involucrine, du polypeptide amyloïde des îlots, de la kératine, cytosquelettique 1 de type II (mélange de kératine-1, -10), du récepteur 1 de l'acide hyaluronique des cellules endothéliales des vaisseaux lymphatiques, de la métalloélastase du macrophage, du complexe métalloprotéinase-9 matricielle:inhibiteur de métalloprotéinase-2, de l'inhibiteur de métalloprotéinease-3, de la protéine de réponse primaire de différenciation myéloïde MyD88, de la nétrine-1, de la nétrine-4, de la molécule d'adhérence cellulaire neuronale, de l'inhibiteur alpha de NF-Kappa-B, du nidogène-1, de l'ostéocalcine, de la pappalysine-1, de la poly [ADP-ribose] polymérase 1 (clivée), de la probêtacelluline, de l'antigène spécifique de la prostate, de la phosphatase acide prostatique, de l'homologue de la protéine NOV, du pro-facteur de croissance transformant alpha, du ligand glycoprotéine 1 pour la sélectine P, de la globuline de liaison aux hormones sexuelles, de FLT3LG, de la ténascine, de la thrombospondine-2, de la lymphopoïétine stromale thymique, de la glycoprotéine transmembranaire NMB, du facteur en trèfle-3, de l'antigène de la néphrite tubulointerstitielle, du membre-lOB de la superfamille des récepteurs du facteur de nécrose tumorale, du membre-8 de la superfamille des récepteurs du facteur de nécrose tumorale, de la protéine Versican du core, de la protéine morphogénétique osseuse de type 7, de l'anhydrase carbonique 9, de la caspase 9, de la collagénase 3, de la Granzyme M, du facteur de croissance EGF-like se liant à l'héparine, du substrat-1 du récepteur à l'insuline, de la kératine, cytosquelettique 6 de type II (mélange de kératine 6A, -6B, - 6C), de la protéine de réponse primaire de différenciation myéloïde MyD88, de FLT3LG, du facteur de croissance endothélial vasculaire D, du récepteur-2 du facteur de croissance endothélial vasculaire, et du récepteur-3 du facteur de croissance endothélial vasculaire sur un échantillon de fluide corporel obtenu sur le sujet pour le procédé de diagnostic ou sur un échantillon d'urine pour le procédé d'évaluation du risque pronostique afin de produire un ou plusieurs résultats d'analyse ; et
la comparaison du ou des résultats d'analyse sous la forme d'une concentration de protéine 7 de liaison au facteur de croissance insuline-like à un seuil prédéterminé sélectionné pour indiquer la présence ou l'absence d'un SRIS, la présence ou l'absence d'un sepsis, la présence ou l'absence d'un sepsis sévère, la présence ou l'absence d'un choc septique, la présence ou l'absence d'un SDMV, ou la comparaison du ou des résultats d'analyse sous la forme d'une concentration de protéine 7 de liaison au facteur de croissance insuline-like à un seuil prédéterminé sélectionné pour indiquer le risque pronostique d'une ou plusieurs issues cliniques, dans lequel lesdites une ou plusieurs issues cliniques comprennent un risque pronostique de mortalité pour le sujet souffrant ou dont on pense qu'il souffre d'un SRIS, d'un sepsis, d'un sepsis sévère, d'un choc septique, ou d'un SDMV.

2. Procédé selon la revendication 1, dans lequel ladite étape de mise en correspondance comprend la comparaison du ou de chacun des résultat(s) desdites une ou plusieurs analyses immunologiques à un niveau de seuil prédéterminé correspondant, sélectionné pour produire une sensibilité ou une spécificité d'au moins 0,7 pour le diagnostic de sepsis, comparativement à un SRIS n'ayant pas évolué en sepsis.

3. Procédé selon la revendication 1, dans lequel ladite étape de mise en correspondance comprend la comparaison du ou de chacun des résultat(s) desdites une ou plusieurs analyses immunologiques à un niveau de seuil prédéterminé correspondant, sélectionné pour produire une sensibilité ou une spécificité d'au moins 0,7 pour le diagnostic de sepsis sévère, comparativement à un SRIS n'ayant pas évolué en sepsis sévère.

4. Procédé selon la revendication 1, dans lequel ladite étape de mise en correspondance comprend la comparaison du ou de chacun des résultat(s) desdites une ou plusieurs analyses immunologiques à un niveau de seuil prédéterminé correspondant, sélectionné pour produire une sensibilité ou une spécificité d'au moins 0,7 pour le diagnostic de choc septique, comparativement à un SRIS n'ayant pas évolué en choc septique.

5. Procédé selon la revendication 1, dans lequel ladite étape de mise en correspondance comprend la comparaison du ou de chacun des résultat(s) desdites une ou plusieurs analyses immunologiques à un niveau de seuil prédéterminé correspondant, sélectionné pour produire un rapport de cotes d'au moins 2 pour le risque pronostique de mortalité.

6. Procédé selon la revendication 1, dans lequel ladite étape de mise en correspondance comprend la comparaison du ou de chacun des résultat(s) desdites une ou plusieurs analyses immunologiques à un niveau de seuil prédéterminé correspondant, sélectionné pour produire un rapport de cotes d'au moins 2 pour le risque pronostique d'aggravation avec évolution d'un sepsis en sepsis sévère ; d'un sepsis ou d'un sepsis sévère en choc septique ; et d'un sepsis, d'un sepsis sévère ou d'un choc septique en SDMV.

7. Procédé selon la revendication 1, dans lequel l'échantillon de fluide corporel est sélectionné dans le groupe constitué du sang, du sérum et du plasma, ou le procédé étant un procédé pronostique, et le procédé établissant une différence entre un risque de sepsis à l'avenir et un risque de sepsis sévère ou de choc septique à l'avenir.

8. Procédé selon la revendication 1, le procédé étant un procédé pronostique, et le procédé établissant une différence entre un risque de sepsis ou de sepsis sévère à l'avenir et un risque de choc septique à l'avenir, ou le procédé étant un procédé diagnostique, et le procédé établissant une différence entre un diagnostic de sepsis et un diagnostic de sepsis sévère ou de choc septique, ou
le procédé étant un procédé diagnostique, et le procédé établissant une différence entre un diagnostic de sepsis ou de sepsis sévère et un diagnostic de choc septique.

9. Procédé in vitro d'évaluation des niveaux de biomarqueurs dans un échantillon de fluide corporel, comprenant :
la réalisation, sur un échantillon de fluide corporel obtenu sur un sujet sélectionné pour une évaluation sur la base d'une détermination indiquant que le sujet risque, au moment présent ou à l'avenir, de recevoir un diagnostic de sepsis, de sepsis sévère, de choc septique ou de SDMV, d'une ou plusieurs analyses de liaison d'analytes, conçues pour détecter la protéine 7 de liaison au facteur de croissance insuline-like, et optionnellement en outre un ou plusieurs biomarqueurs sélectionnés dans le groupe constitué de la macroglobuline alpha-2, de l'angiogénine, du récepteur de l'angiopoïétine-1, de la protéine 3 apparentée à l'angiopoïétine, de la protéine 4 apparentée à l'angiopoïétine, de la protéine 6 apparentée à l'angiopoïétine, de l'apolipoprotéine A-II, de l'apolipoprotéine C-III, de l'apolipoprotéine(a), de la protéine morphogénétique osseuse de type 7, de la cadhérine-16, de la cadhérine-3, de l'antigène de cancer 15-3, de l'antigène de cancer 19-9, de l'anhydrase carbonique 9, de la molécule d'adhésion cellulaire 1 liée à l'antigène carcino-embryonnaire, de la molécule d'adhésion cellulaire 5 liée à l'antigène carcino-embryonnaire, de la caspase-3, active, de la cathepsine S, de la chimiokine 1 à motif C-C, de la chimiokine 15 à motif C-C, de la chimiokine 17 à motif C-C, de la chimiokine 18 à motif C-C, de la chimiokine 20 à motif C-C, de la chimiokine 21 à motif C-C, de la chimiokine 24 à motif C-C, de la clustérine, de la collagénase 3, du peptide C (insuline), de la chimiokine 11 à motif C-X-C, de la chimiokine 16 à motif C-X-C, de l'inhibiteur de kinase cycline-dépendante 1, de la protéine 1 contenant le domaine DDRGK, de l'endostatine, du récepteur du facteur de croissance épidermique, de l'épiréguline, de la molécule d'adhérence des cellules épithéliales, du récepteur de l'érythropoïétine, du facteur de croissance fibroblastique de type 19, du facteur de croissance fibroblastique de type 23, de la galectine 3, du polypeptide inhibiteur gastrique, du glucagon, du glucagon-like peptide 1, de la glutathion S-transférase P, de la protéine du choc thermique bêta-1, du facteur de croissance se liant à l'héparine de type 1, du récepteur cellulaire 1 du virus de l'hépatite A, du récepteur du facteur de croissance des hépatocytes, de l'insuline, du récepteur du facteur de croissance 1 insuline-like, de la protéine 6 de liaison au facteur de croissance insuline-like, de l'interféron alpha-2, de l'interleukine-20, de l'interleukine-21, de l'interleukine-28A, de l'interleukine-29, de l'interleukine-33, de l'involucrine, du polypeptide amyloïde des îlots, de la kératine, cytosquelettique 1 de type II (mélange de kératine-1, -10), du récepteur 1 de l'acide hyaluronique des cellules endothéliales des vaisseaux lymphatiques, de la métalloélastase du macrophage, du complexe métalloprotéinase-9 matricielle:inhibiteur de métalloprotéinase-2, de l'inhibiteur de métalloprotéinease-3, de la protéine de réponse primaire de différenciation myéloïde MyD88, de la nétrine-1, de la nétrine-4, de la molécule d'adhérence cellulaire neuronale, de l'inhibiteur alpha de NF-Kappa-B, du nidogène-1, de l'ostéocalcine, de la pappalysine-1, de la poly [ADP-ribose] polymérase 1 (clivée), de la probêtacelluline, de l'antigène spécifique de la prostate, de la phosphatase acide prostatique, de l'homologue de la protéine NOV, du pro-facteur de croissance transformant alpha, du ligand glycoprotéine 1 pour la sélectine P, de la globuline de liaison aux hormones sexuelles, de FLT3LG, de la ténascine, de la thrombospondine-2, de la lymphopoïétine stromale thymique, de la glycoprotéine transmembranaire NMB, du facteur en trèfle-3, de l'antigène de la néphrite tubulointerstitielle, du membre-lOB de la superfamille des récepteurs du facteur de nécrose tumorale, du membre-8 de la superfamille des récepteurs du facteur de nécrose tumorale, de la protéine Versican du core, de la protéine morphogénétique osseuse de type 7, de l'anhydrase carbonique 9, de la caspase 9, de la collagénase 3, de la Granzyme M, du facteur de croissance EGF-like se liant à l'héparine, du substrat-1 du récepteur à l'insuline, de la kératine, cytosquelettique 6 de type II (mélange de kératine 6A, -6B, - 6C), de la protéine de réponse primaire de différenciation myéloïde MyD88, de FLT3LG, du facteur de croissance endothélial vasculaire D, du récepteur-2 du facteur de croissance endothélial vasculaire, et du récepteur-3 du facteur de croissance endothélial vasculaire en introduisant l'échantillon de fluide corporel obtenu sur le sujet dans un instrument d'analyse qui (i) entre en contact avec une pluralité de réactifs qui se lient spécifiquement pour la détection de la pluralité de biomarqueurs avec l'échantillon d'urine, et (ii) produit un ou plusieurs résultats d'analyse indiquant la liaison de chaque biomarqueur qui est analysé à un réactif de liaison spécifique respectif dans la pluralité de réactifs ;
la comparaison du ou des résultats d'analyse sous la forme d'une concentration de protéine 7 de liaison au facteur de croissance insuline-like à un seuil prédéterminé sélectionné pour indiquer un risque, à l'avenir ou au moment présent, de recevoir un diagnostic de sepsis, de sepsis sévère, de choc septique ou de SDMV, et
l'affichage desdits un ou plusieurs résultats d'analyse provenant de l'instrument d'analyse en tant que résultat quantitatif sous une forme lisible par l'homme.

10. Procédé selon la revendication 9, dans lequel le sujet est sélectionné pour une évaluation sur la base d'une détermination indiquant que le sujet présente un sepsis et risque à l'avenir de recevoir un diagnostic de sepsis sévère, de choc septique ou de SDMV, ou
dans lequel le sujet est sélectionné pour une évaluation sur la base d'une détermination indiquant que le sujet présente un sepsis ou un sepsis sévère et risque à l'avenir de recevoir un diagnostic de choc septique ou de SDMV, ou
dans lequel une pluralité de résultats d'analyse sont combinés en utilisant une fonction qui convertit lesdits résultats d'analyse en un seul résultat composite, ou
dans lequel le sujet est sélectionné pour une évaluation sur la base d'une détermination indiquant que le sujet risque à l'avenir de recevoir un diagnostic de sepsis sévère, de choc septique ou de SDMV au cours d'une période sélectionnée dans le groupe constitué de 21 jours, 14 jours, 7 jours, 5 jours, 96 heures, 72 heures, 48 heures, 36 heures, 24 heures et 12 heures, ou
dans lequel le sujet est sélectionné pour une évaluation sur la base d'une détermination indiquant que le sujet risque à l'avenir de recevoir un diagnostic d'une ou plusieurs d'une lésion de la fonction rénale, d'une fonction rénale réduite, d'une amélioration de la fonction rénale, et d'une insuffisance rénale aiguë (IRA) future, ou
dans lequel la pluralité d'analyses sont des analyses immunologiques réalisées en (i) introduisant l'échantillon d'urine dans un dispositif d'analyse comprenant une pluralité d'anticorps, dont au moins un se lie à chaque biomarqueur qui est analysé, et (ii) en produisant un résultat d'analyse indiquant la liaison de chaque biomarqueur à son anticorps respectif.
